# EUROPEAN PATENT SPECIFICATION

(11) **EP 3 503 880 B1**
(45) Date of publication and mention of the grant of the patent: **27.12.2023**
(21) Application number: 17768011.3
(22) Date of filing: 29.08.2017
(51) Int. Cl.: A61K 31/00, A61K 31/685, A61P 25/00

(54) **COMPOUNDS FOR TREATMENT OF INTRACTABLE EPILEPSY AND DOORS SYNDROME**
VERBINDUNGEN ZUR BEHANDLUNG VON THERAPIEREFRAKTÄRER EPILEPSIE UND DOORS-SYNDROM
COMPOSÉS POUR LE TRAITEMENT DE L'ÉPILEPSIE RÉFRACTAIRE ET DU SYNDROME DOORS

(30) Priority: 29.08.2016 GB 201614609
(43) Date of publication of application: 03.07.2019
(73) Proprietor: VIB VZW, 9052 Gent (BE); Katholieke Universiteit Leuven K.U. Leuven R&D, 3000 Leuven (BE); Vrije Universiteit Brussel, 1050 Brussel (BE)
(72) Inventor: VERSTREKEN, Patrik, 3052 Blanden (BE); LÜTHY, Kevin, 3000 Leuven (BE); VERSÉES, Wim, 1785 Merchtem (BE); FISCHER, Baptiste, 1170 Watermaal-Bosvoorde (BE); PAESMANS, Jone, 1600 Sint-Pieters-Leeuw (BE)
(86) International application number: PCT/EP2017/071622
(87) International publication number: WO 2018/041812

(56) References cited:
- Simona Balestrini ET AL: "TBC1D24 genotype-phenotype correlation Epilepsies and other neurologic features", Neurology, 8 June 2016 (2016-06-08), pages 77-85, XP55415984, DOI: 10.1212/WNL.0000000000002807 Retrieved from the Internet: URL:http://www.neurology.org/content/87/1/ 77.full.pdf#page=1&view=FitH [retrieved on 2017-10-16]
- C. J. STEFAN ET AL: "The Yeast Synaptojanin-like Proteins Control the Cellular Distribution of Phosphatidylinositol (4,5)-Bisphosphate", MOLECULAR BIOLOGY OF THE CELL, vol. 13, no. 2, 1 February 2002 (2002-02-01), pages 542-557, XP55416084, US ISSN: 1059-1524, DOI: 10.1091/mbc.01-10-0476
- BAPTISTE FISCHER ET AL: "Skywalker-TBC1D24 has a lipid-binding pocket mutated in epilepsy and required for synaptic function", NAT. STRUCT. MOL. BIOL., vol. 23, no. 11, 26 September 2016 (2016-09-26), pages 965-973, XP55415981, ISSN: 1545-9993, DOI: 10.1038/nsmb.3297

## Description

### FIELD OF THE INVENTION

The present invention relates to the field of neurodegenerative diseases, and the prevention and/or treatment of TBC1 D24-associated disorders, such as DOORS syndrome, (intractable) epilepsy, and nonsyndromic deafness. In particular, the present invention relates to phosphoinositide phosphatase inhibitors and screening methods for producing such inhibitors, more particularly, Synaptojanin-1 5' phosphatase inhibitors, to increase phosphatidylinositol 4,5-bisphosphate (PI(4,5)P₂) levels in neuronal cells.

### BACKGROUND

*TBC1D24* encodes a protein with an N-terminal TBC (Tre2/Bub2/Cdc16) domain linked to a TLDc (TBC/LysM) domain. This combination of a TBC domain and a TLDc domain is unique amongst human proteins¹⁵, but orthologues with the same domain organization are found in many other species including *Drosophila melanogaster* where the gene is called *skywalker* (*sky*)*.* Mutations in *TBC1D24* cause early onset epilepsy accompanied with variable degrees of intellectual disability¹⁻⁶, hearing loss⁷⁻¹⁰ and cortical myoclonus and cerebellar ataxia¹¹. In addition, DOORS syndrome (deafness, onychodystrophy, osteodystrophy, mental retardation and seizures) is attributed to mutations in *TBC1D24*^{12,13}*.* Most mutations are recessive loss of function mutations¹⁴, but how these mutations affect protein activity and neuronal function is unknown. Fruit flies with such pathogenic mutations also show severe neurological defects, including impaired synaptic vesicle trafficking and seizures.

Although TBC domains typically interact with small GTPases that regulate vesicle trafficking¹⁶, TBC1 D24 and Skywalker contain an atypical TBC domain that lacks the arginine and glutamine residues¹⁵ required for stimulating GTPase activity, suggesting that they might use an alternative mechanism to catalyze GTP hydrolysis²⁰. Currently, the knowledge is lacking to determine how pathogenic mutations affect TBC1 D24 function.

Phosphatidylinositol (PI) signaling is tightly regulated by a number of enzymes, such as kinases, phosphatases, and phospholipases. In the central nervous system, the levels of Pls in nerve terminals are regulated by specific synaptic phosphatidylinositol phosphate phosphatases, such as synaptojanin 1 (SYNJ1), but also kinases. Within the field of intractable epilepsy, mutations in the catalytic domain of phosphoinositide 3-kinases (*PIK3ca* mutations) were shown to cause pathological effects, meaning that PI3K inhibitors envisage a new avenue for anti-epileptic therapy⁵⁹. Such kinase inhibitors potentially also impact the PI(4,5)P₂ levels, and therefore a PI3K inhibitor could further increase the possibilities for treatment of refractory epilepsy.

Synaptojanin-1 encodes a polyphosphatidylinositol phosphatase containing two consecutive phosphatase domains, and plays a role in neuronal synaptic vesicle dynamics. The one phosphatase domain Sacl was previously functionally linked to parkinsonism, and the reduction of the dual phosphatase activity of both domains was shown to underlie severe neonatal refractory epilepsy⁶⁰.

It would be advantageous to unravel the TBC1 D24 structure to search for treatments that rescue the defects caused by the pathological *TBC1D24* mutations, as this could lead to therapeutic solutions for many TBC1 D24-associated disorders such as intractable epilepsy, DOORS syndrome and nonsyndromic deafness.

### SUMMARY OF THE INVENTION

The invention is based on the unravelling of the crystal structure of the TBC domain of Skywalker, which revealed an unusual positively charged pocket that is highly conserved among TBC1 D24 orthologues but is absent in other known TBC domains. The most prevalent and severe forms of TBC1 D24-induced neurological disease are also caused by mutations that map to this pocket. Surprisingly, this pocket allowed the TBC domain to bind directly to membranes containing phosphoinositides phosphorylated in the 4 and 5 position (e.g. PI(4,5)P₂, PI(3,4,5)P₃). In presynaptic terminals the pocket mediates binding to the membrane, which is required for normal Skywalker function and restriction of Skywalker mobility. Indeed, pathogenic DOORS syndrome mutations or mutations that neutralize or revert positive charge in the phosphoinositide-binding pocket of Skywalker reduce phosphoinositide binding and cause presynaptic vesicle trafficking defects. In adult fruit flies they elicit seizures and further neurological defects. Interestingly, genetically increasing phosphatidylinositol 4,5-bisphosphate (PI(4,5)P₂), by mutating one copy of the synaptic phosphoinositide phosphatase Synaptojanin^{25,26}, strongly suppressed all the cellular and neurological defects in adult Skywalker cationic-pocket-mutant flies. Hence, identification of compounds which increase PI(4,5)P₂ and/or PI(3,4,5)P₃ levels, especially in neurons, allows for therapeutic solutions for many TBC1 D24-associated disorders such as intractable epilepsy, DOORS syndrome and nonsyndromic deafness. Furthermore, besides acting on the PI(4,5)P₂ and/or PI(3,4,5)P₃ levels via manipulation of its phosphorylation and dephosphorylation or phosphatase events, one could also affect the hydrolysis in the brain, which occurs in response to stimulation of a large number of receptors via two major signaling pathways thereby providing an alternative targeting opportunity, more specifically: a) the activation of G-protein linked neurotransmitter receptors (e.g. glutamate and acetylcholine), mediated by phospholipase C β (PLCβ), and b) the activation of tyrosine kinase linked receptors for growth factors and neurotrophins (e.g. NGF, BDNF), mediated by PLCγ.

The invention further relates to the identification of phosphoinositide phosphatase inhibitors with the capability to increase PI(4,5)P₂ and/or PI(3,4,5)P₃ levels as therapeutic candidates for treatment of TBC1 D24-associated disorders.

In a first aspect, the invention relates to phosphoinositide phosphatase inhibitors leading to increased PI(4,5)P₂ levels in a cell, for use in treatment of TBC1 D24-associated disorders. In one embodiment, the TBC1 D24-associated disorders, comprise severe forms of epilepsy (early onset epilepsy, familial infantile myoclonic epilepsy (FIME), focal epilepsy, dysarthria, myoclonic epilepsy with dystonia, familial malignant migrating partial seizures of infancy, *De Flippo* malignant migrating partial seizures of infancy), variable degrees of intellectual disability, hearing loss (nonsyndromic deafness, autosomal-dominant nonsyndromic hearing loss, and dominant nonsyndromic hearing impairment), cortical myoclonus and cerebellar ataxia, and DOORS syndrome (deafness, onychodystrophy, osteodystrophy, mental retardation and seizures).

In a particular aspect, said phosphoinositide phosphatase is Synaptojanin-1, and said inhibitor for use in treatment of TBC1D24-associated disorders, is a Synaptojanin-1 phosphatase inhibitor. In another particular embodiment, said inhibitors or compounds for use in treatment of TBC1D24-associated disorders are Synaptojanin-1 5' phosphatase domain phosphatase inhibitors. In another embodiment, said phosphoinositide phosphatase inhibitors increase PI(4,5)P₂ levels in neuronal cells.

One aspect relates to the invention wherein said phosphoinositide (PI) phosphatase inhibitors, for use in treatment of TBC1D24-associated disorders, are selected from a list of inhibitors consisting of bpV(pic), perifosine, edelfosine, miltefosine, erufosine, or YU144118, or a salt or hydrate derivative form thereof.

In a specific aspect of the invention, the PI phosphatase inhibitor for use in treatment of TBC1D24-associated disorders is an alkyl phospholipid. A more particular embodiment relates to an alkyl phospholipid, for use in treatment of TBC1D24-associated disorders, wherein said alkyl phospholipid is miltefosine, or a salt or hydrate derivative form thereof. Alternatively, embodiments are provided wherein said alkyl phospholipid is perifosine, edelfosine, erufosine, or a salt or hydrate derivative form thereof. Alternative embodiments relate to the phosphoinositide phosphatase inhibitor, for use in treatment of TBC1D24-associated disorders, wherein said inhibitor is YU144118, or a salt or hydrate derivative form thereof.

### DESCRIPTION OF THE FIGURES

The drawings described are only schematic and are non-limiting. In the drawings, the size of some of the elements may be exaggerated and not drawn on scale for illustrative purposes.

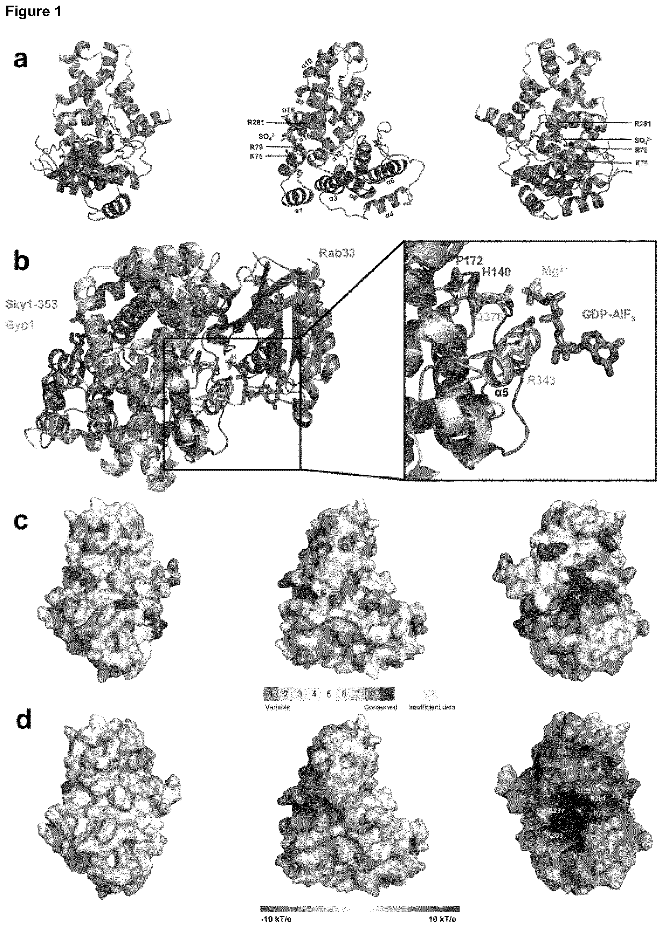

Figure 1. Crystal structure of the unconventional TBC domain of Skywalker (Sky1-353)
   (**a**) Cartoon representation of the structure of Sky1-353 in three different orientations related by a 90° rotation around a vertical axis. The left and right panels show a view on the presumed GTPase binding surface and the surface containing the cationic pocket, respectively. In the middle panel the helices are numbered in accordance with the numbering used in Gyp1¹⁹. Helices of the N- and C-terminal lobe of the TBC domain are indicated in dark and light green respectively. A sulfate ion that is bound to the cationic pocket is shown as sticks, together with the interacting residues K75, R79 and R281.
   (**b**) Superposition of Sky1-353 onto the Gyp1-Rab33-GDP.AIF3 complex. Sky1-353 is colored as in (**a**), the TBC domain of Gyp1 is colored light grey and Rab33 dark grey. GDP.AIF3 bound to Rab33 is shown as sticks. The right panel shows a close-up of the boxed region, Rab33 is omitted for clarity. Helix α5 of Gyp1 is replaced by a long loop region in Sky. The catalytic arginine and glutamine fingers of Gyp1 (R343 and Q378) correspond to a histidine and proline in the Sky sequence (H140 and P172).
   (**c**) Surface representation of Sky1-353 shown in the same orientation as in (**a**) with conservation scores of residues mapped on the surface. The right panel shows a region of high conservation surrounding the bound sulfate ion.
   (**d**) Electrostatic surface representation of Sky1-353 shown in the same orientation as in (**a**). The residues constituting the cationic pocket surrounding the bound sulfate ion are indicated.

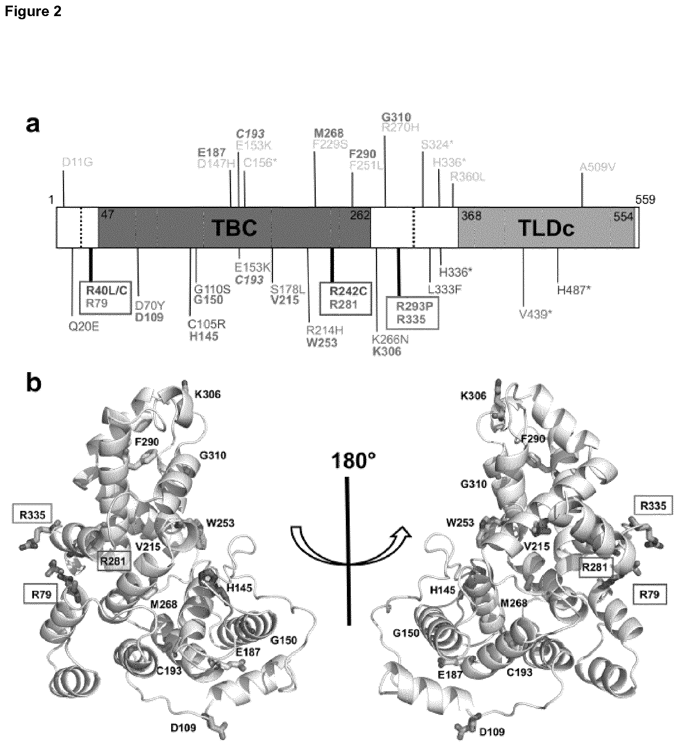

Figure 2: Disease -associated mutations in TBC1D24/Sky
   (**a**) Domain organization of TBC1D24/Sky and the position of the mutations implicated in various epilepsy syndromes (in yellow), DOORS syndrome (in red) and non-syndromic deafness (in green). Nonsense mutations are indicated by *. The corresponding residue numbers in Sky are indicated in grey. The three arginine mutants present in the cationic phosphoinositide-binding pocket are highlighted. The indicated TBC and TLDc domains correspond to the core domain prediction by Pfam. The dotted lines indicate the boundaries of the peptide visible in our Sky1-353 crystal structure.
   (**b**) Localization of residues corresponding to pathological mutations on the Sky1-353 crystal structure. The residues are color-coded according to the associated disease phenotype as in (**a**). The three arginine residues present in the cationic phosphoinositide-binding pocket are highlighted.

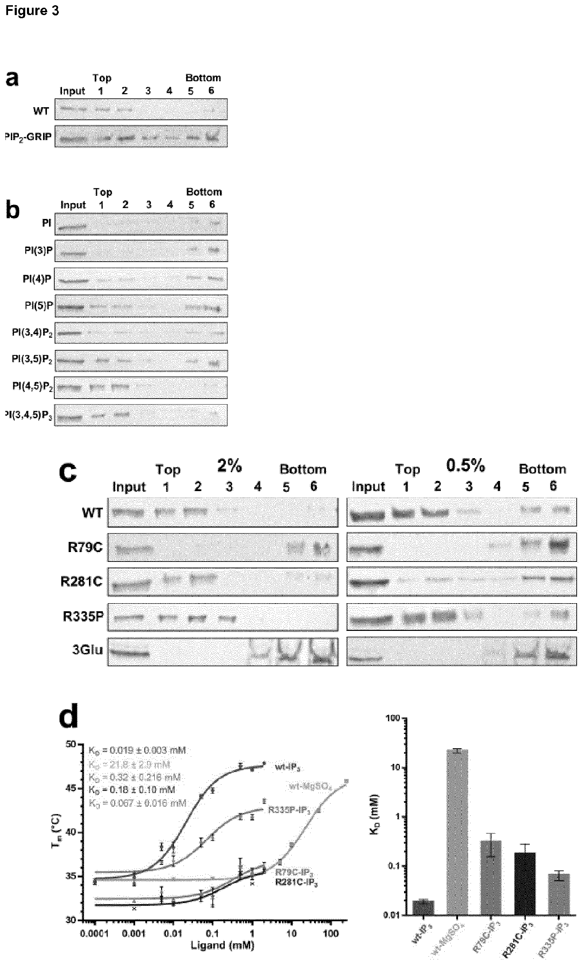

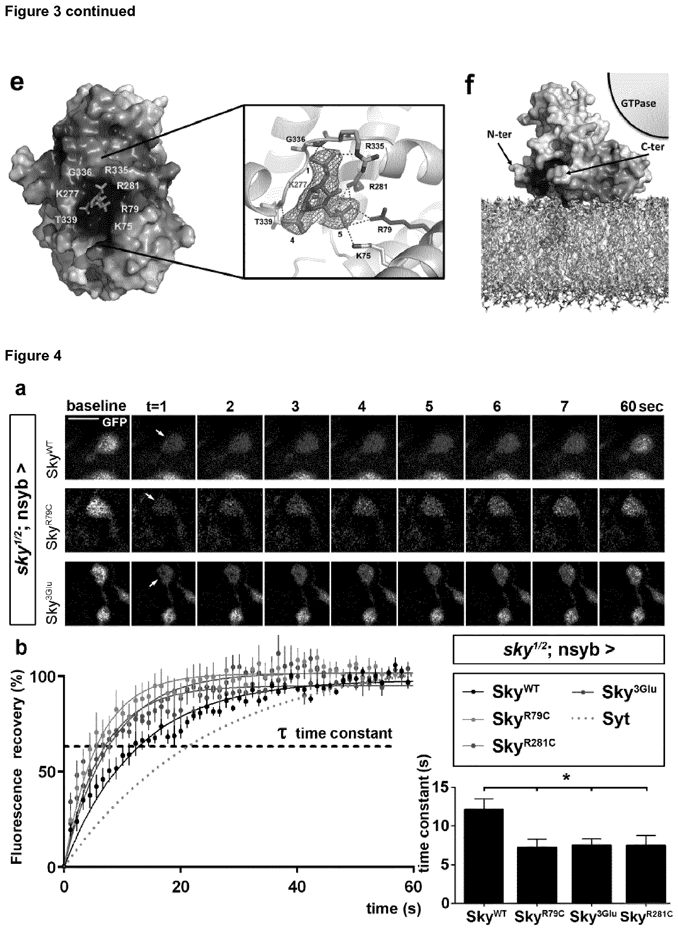

Figure 3: The cationic pocket on the TBC domain of Sky binds phosphoinositides
   (**a**) Western blots of liposome flotation assays using wild type Sky1-353 (WT) or PI(4,5)P₂-GRIP (a positive control) and Folch lipid liposomes. The input and 6 fractions (after centrifugation) from the top (1) to the bottom (6) are loaded on a SDS-PAGE gel and revealed by Western Blot. The liposomes and associated proteins are present in the top fractions. (N=2 independent experiments).
   (**b**) Western blots of liposome flotation assays using wild type Sky1-353 and liposomes (PC:PS) enriched with 2% of the indicated phosphoinositide. (N=2 independent experiments).
   (**c**) Western blots of liposome flotation assays using wild type or mutant (R79C, R281C, R335P, 3Glu) Sky1-353 and liposomes enriched with 2% (left panel) or 0.5% (right panel) of PI(4,5)P₂. (N=2 independent experiments).
   (**d**) Titration curves for binding of IP3 to wild type and mutant Sky1-353 (mean ± s.d.). Melting temperatures (Tm) at different ligand concentrations were obtained via thermal shift assays. Plotting the Tm values versus ligand concentration yields binding curves that were fitted on a quadratic equation to obtain KD values. Curves for IP3 binding to wild type Sky1-353 and to the disease-associated mutations R79C, R281C and R335P are shown. The curve for MgSO4 binding to wild type Sky1-353 is shown as a reference. N=3. Right, the calculated KD values.
   (**e**) Crystal structure of Sky1-353 bound to IP3, the head group of PI(4,5)P₂. Electrostatic surface representation of Sky1-353 in complex with IP3 (red, negative; blue, positive). Residues that interact with IP3 are indicated. The box represents a close-up of the IP3 binding site. IP3 is represented in dark grey sticks with the 2Fo-Fc electron density map (at 1.3 σ) shown as a blue mesh. The interacting residues are shown as sticks with residues that are mutated in DOORS syndrome and nonsyndromic deafness colored red and green, respectively.
   (**f**) Indicative docking model for binding of Sky1-353 to a PI(4,5)P₂-containing biological membrane. A POPC (1-palmitoyl-2-oleoyl-sn-glycero-3-phosphocholine) bilayer containing a molecule of PI(4,5)P₂ was generated and the Sky1-353-IP3 crystal structure was docked on this membrane by superimposing the protein-bound IP3 group on the membrane-embedded PI(4,5)P₂. The presumed GTPase-interaction surface of Sky1-353 is indicated.
Figure 4: Mutations in the cationic pocket increase Sky protein diffusion in synaptic terminals.
   (**a**) Images of neuromuscular junction synaptic boutons during a time course experiment where wild type and mutant GFP-Sky (SkyWT, SkyR79C, SkyR281C and Sky3Glu) in the indicated boutons was bleached (arrow, t=1) and where the recovery of fluorescence was then followed for the next minute (t=2-60 s) (FRAP). Wild type and mutant GFP-Sky are all expressed using nSybGal4 in *sky1*/*2* mutants (*yw eyFLP; FRT40A sky1:FRT40A sky2*)*.*
   (**b**) Recovery of fluorescence levels in the bleached bouton area starting at the time point following bleaching and normalized to maximum recovery levels. The solid line indicates a single exponential fit of the recovery kinetics and the horizontal dotted line indicates 63.2% recovery of fluorescence. The grey dotted line is a single exponential fit of the fluorescence recovery following the bleaching of Synaptotagmin-GFP (data points are not shown separately), a synaptic vesicle membrane associated protein that we used as a control for a 100% membrane associated protein. The average time constants τ of fluorescence recovery are shown on the right. N=8. Scale bar: 2 µm. Error bars: SEM. ANOVA, Dunnett: p= 0.014 (*).

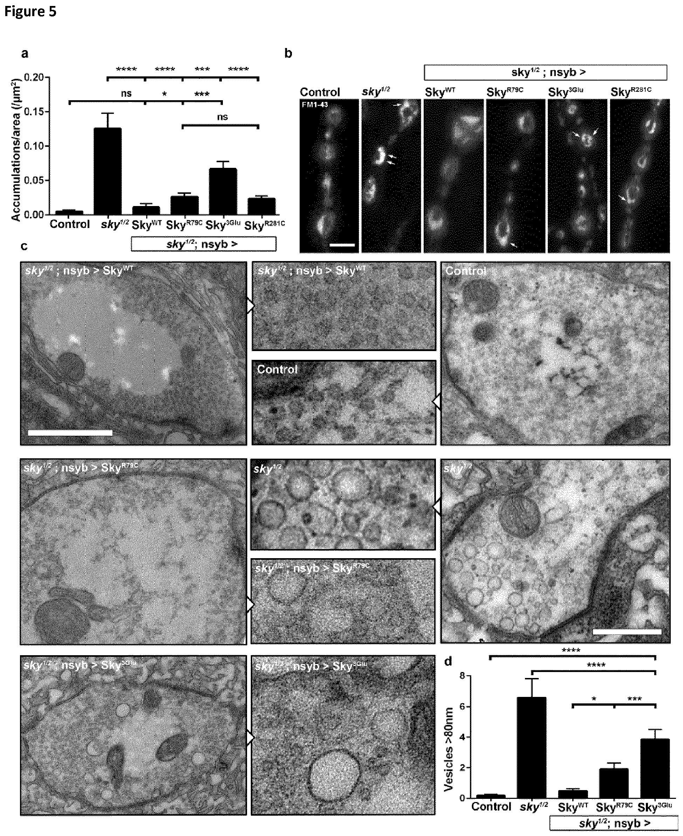

Figure 5: The cationic pocket in Sky is required for presynaptic function
   (**a, b**) Quantification of the number of FM 1-43 accumulations per boutonic area and images of FM 1-43 labeling (arrows indicate FM 1-43 accumulations) at third instar larval synaptic boutons in control animals (yw *eyFLP; FRT40A*) and in *sky1*/*2* mutants (*yw eyFLP; FRT40A sky1:FRT40Asky2*) as well as in *sky1*/*2* animals expressing wild type or mutant GFP-Sky (as indicated) using nSybGal4. N=10, 5 animals with 2 NMJs per animal. Scale bar: 2 µm. Error bars: SEM. ANOVA, Dunnett: p< 0.0001 (****); ns, not significant, p< 0.05 (*), p< 0.001 (***), p< 0.0001(****). t test (SkyR79C vs SkyR281C): ns, not significant
   (**c**) Transmission electron micrographs of larval synaptic boutons of the following genotypes: *sky1*/*2* animals (*yw eyFLP; FRT40A sky1:FRT40A sky2*) expressing wild type Sky (SkyWT) using nSybGal4 (nsyb>); control animals (*yw eyFLP; FRT40A); sky1*/*2* mutants expressing mutant Sky (SkyR79C or Sky3Glu) using nSybGal4 and *sky1*/*2* mutants. The insets indicate the synaptic vesicles and larger endosomal/cisternal-like structures.
   (**d**) Quantification of the number of large-diameter vesicles/endosomes (>80 nm) per boutonic area. Scale bar: 1 µm as indicated for SkyWT, and 0.5 µm for *sky1*/*2.* N=8 (control, sky1/2 and Sky3Glu), N=13 (SkyWT) and N=15 (SkyR79C) bouton profiles from >5 animals. Error bars: SEM. ANOVA, Dunnett: p< 0.0001 (****); p< 0.05 (*), p< 0.001 (***), p< 0.0001 (****).

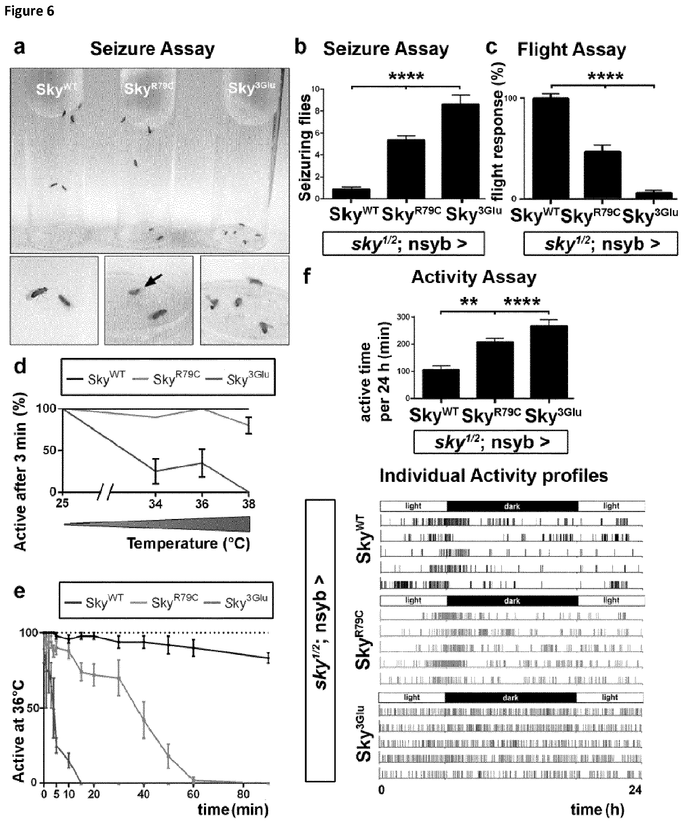

Figure 6: Mutation of the cationic pocket of Sky induces susceptibility to seizures, loss of coordination and hyperactivity in adult animals.
   (**a, b**) Photograph of adult *sky1*/*2 Drosophila* males (*yweyFLP*/*Y; FRT40A sky1:FRT40A sky2*) expressing GFP-SkyWT, GFP-SkyR79C or GFP-Sky3Glu using nSybGal4, following 10 seconds of vortex stimulation. The insets show the seizuring flies upon expressing GFP-SkyR79C or GFPSky3Glu but not GFP-SkyWT and these data are also quantified in (**b**) as the number of flies that fail to stand within a 5 second period. N=5; Error bars: SEM; ANOVA, Dunnett: p< 0.0001(****).
   (**c**) Quantification of flight response of flies with genotypes indicated in (**a**). *sky1*/*2* flies expressing GFP-SkyR79C or GFP-Sky3Glu fly significantly worse than those expressing GFPSkyWT (nSybGal4). N=8 (5 flies each time). Error bars: SEM; ANOVA, Dunnett: p <0.0001(****).
   (**d, e**) Quantification of the number of flies (genotypes in (**a**)) standing when incubated for 3 min at the indicated temperatures (**d**) or incubated at 36°C for the indicated time (**e**). (**d**) N=40 (SkyWT, Sky3Glu); N=20 (SkyR79C).
   (**e**) N=50 (SkyWT, SkyR79C); N=20 (Sky3Glu). Error bars: SEM.
   (**f**) Quantification (top) of total active time (the number of minutes during which a fly crosses the IR beam at least once) and representation of activity over a 72 h period (bottom) of flies with genotypes indicated in (**a**). Each horizontal line represents the activity of a single fly during a 24 h period (12 h light and 12 h dark) and each vertical line represents an active minute when the fly crossed the IR beam (at least) once. Error bars: SEM; N=30 (SkyWT, SkyR79C); N=15 (Sky3Glu); ANOVA, Dunnett: p< 0.0001; p< 0.01 (**), p< 0.0001 (****).

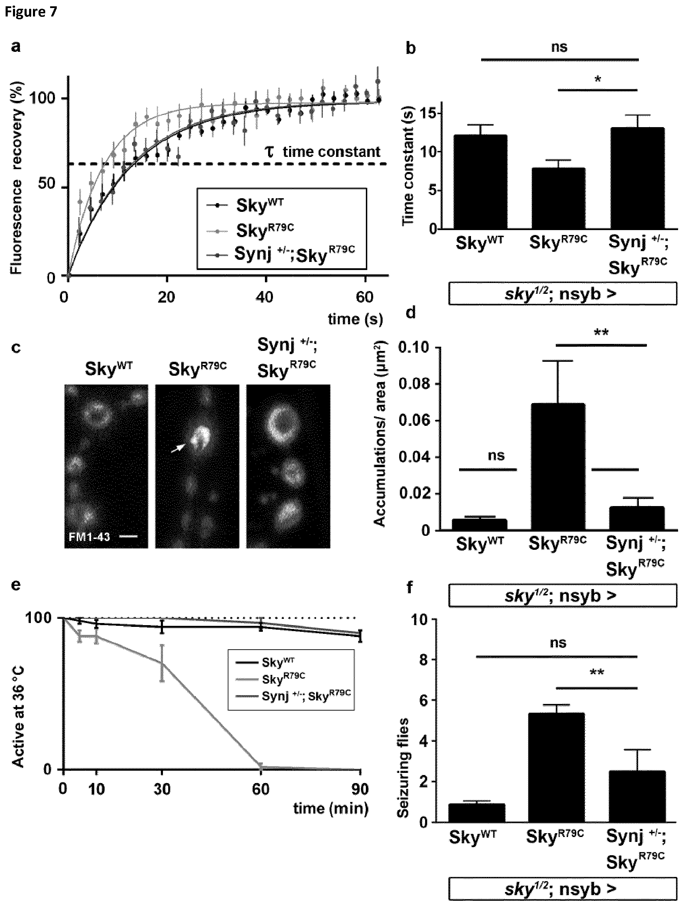

Figure 7: Partial loss of *synaptojanin* rescues Sky with cationic pocket mutations
   (**a**, **b**) Recovery of fluorescence levels of wild type and mutant GFP-Sky in a bleached bouton area starting at the time point following bleaching and normalized to maximum recovery levels (**a**) in the following genotypes: flies expressing GFP-SkyWT using nSybGal4 (SkyWT); flies expressing GFP-SkyR79C using nSybGal4 (SkyR79C) and heterozygous *synaptojanin* mutants (*yweyFLP*/*yw; FRT42D synj1*/+) expressing GFP-SkyR79C using nSybGal4 (*synj*+/-; SkyR79C). The solid line indicates a single exponential fit of the recovery kinetics and the horizontal dotted line indicates 63.2% recovery of fluorescence. The average time constants **τ** of fluorescence recovery are shown on the right (**b**). N=8. Error bars: SEM. t test: ns: not significant, p= 0.017(*).
   (**c, d**) Images of FM 1-43 labeling (**c**) (arrows indicate FM 1-43 accumulations) and quantification of the number of FM 1-43 accumulations per boutonic area (**d**) at third instar larval synaptic boutons in *sky* mutants (*yw eyFLP; FRT40A sky1:FRT40A sky2*) expressing wild type GFP-Sky (using nSybGal4 (SkyWT); in *sky* mutants expressing GFP-SkyR79C using nSybGal4 (SkyR79C) and in *sky* mutants heterozygous for *synaptojanin* (*sky1 synj1*/*FRT40A sky2*) and expressing GFP-SkyR79C using nSybGal4 (synj+/-; SkyR79C). N=10 NMJs from 5 animals. Scale bar: 2 µm. Error bars: SEM. t test: ns: not significant, p= 0.0027 (**).
   (**e**) Quantification of the number of flies (genotypes in (**c**, **d**)) standing when incubated at 36°C for the indicated time. N=40 (SkyWT); N=20 (SkyR79C) and N=30 (synj+/-; SkyR79C). Error bars: SEM.
   (**f**) Quantification of the number of flies that fail to stand within a 5 s period following a 10 s vortex (seizuring flies). Male flies of the genotypes indicated in (**c**, **d**) are included. N=5; Error bars: SEM; t test: ns: not significant, p<0.01 (**).

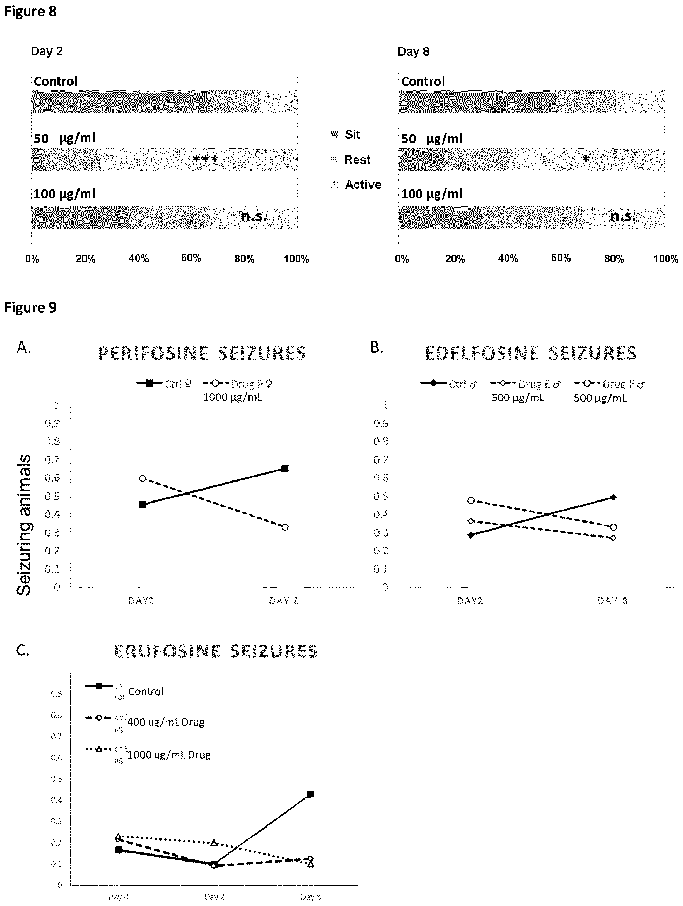

Figure 8: Miltefosine feeding rescues seizure phenotype in Sky with cationic pocket mutations Quantification of seizuring *sky*^{R79C} flies for control or mock-treated flies (upper bar), Miltefosine fed flies at 50 µg/mL (middle bar) or at 100 µg/mL (lower bar) after 2 days of drug feeding (left panel), or 8 days of drug feeding (right panel). The percentage of flies that was active, in rest, or sitting still was analysed for all treatments for n= 20 flies. Treatment with a high, probably toxic, concentration of 100 µg/mL did not lead to significant difference in seizure-behavour (n.s, non-significant) as compared to the control population, whereas a treatment with 50 µg/mL Miltefosine resulted in a significant rescue of seizure-like behaviour with p< 0.001 (***) after 2 days of treatment and p<0.1 (*) still after 8 days of treatment.
Figure 9: Alkyl phospholipid compound treatment to rescue seizure phenotype in Sky with cationic pocket mutations
   Quantification of seizuring *sky*^{R79C} flies for control or mock-treated flies and Drug-treated flies fed with Perifosine (A), Edelfosine (B), and Erufosine (C), for a daily administration at a concentration of 1000 µg/mL Perifosine, 500 µg/mL Edelfosine, and 400 or 1000 µg/mL Erufosine over 8 days of drug feeding. The fraction of seizuring flies was analysed for all treatments for n= 10 flies at each timepoint.

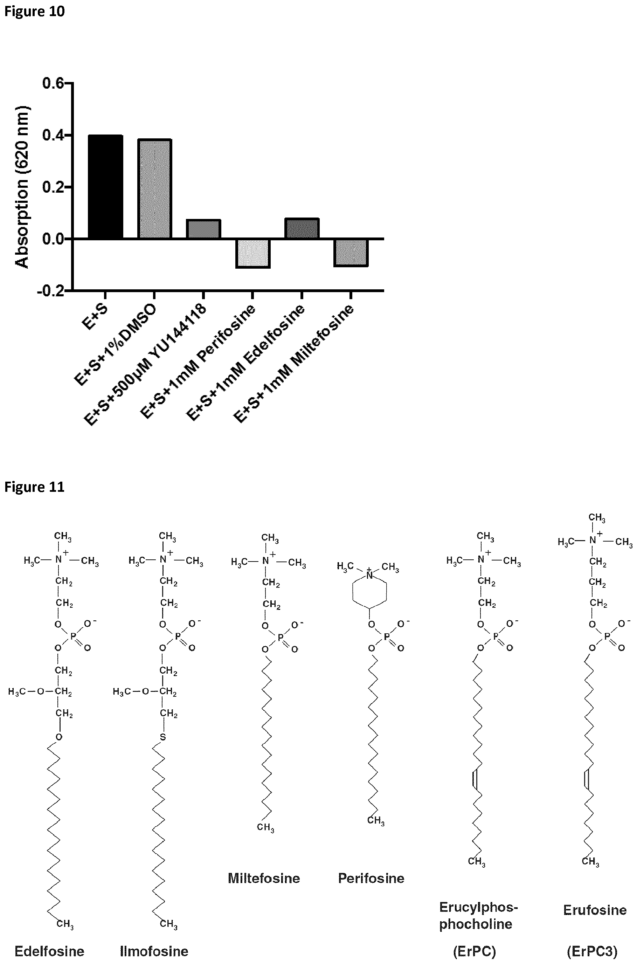

Figure 10: Malachite Green phosphatase assay specific for Synj1 5' phosphatase activity inhibitors.
   The optimized Malachite Green assay was used to test the inhibitory capacity of the compounds, YU144118 as well as of the alkyl phospholipids Miltefosine, Perisofine, and Edelfosine, in the presence of the 5'-phosphatase domain of Synaptojanin-1 and IP₃ as substrate. As compared to the negative (E+S) and DMSO (E+S+1% DMSO) negative controls, the compounds illustrated a complete inhibition of the activity when analysed after 1h of measurement and subtracting of the background signal due to the presence of the compound at time point zero (E=5'-phosphatase domain of Synaptojanin-1; S= IP₃).
Figure 11: Chemical structures of clinically relevant alkyl phospholipids.

### DETAILED DESCRIPTION TO THE INVENTION

The present invention will be described with respect to particular embodiments and with reference to certain drawings but the invention is not limited thereto but only by the claims. Any reference signs in the claims shall not be construed as limiting the scope. Of course, it is to be understood that not necessarily all aspects or advantages may be achieved in accordance with any particular embodiment of the invention. Thus, for example those skilled in the art will recognize that the invention may be embodied or carried out in a manner that achieves or optimizes one advantage or group of advantages as taught herein without necessarily achieving other aspects or advantages as may be taught or suggested herein.

The invention, both as to organization and method of operation, together with features and advantages thereof, may best be understood by reference to the following detailed description when read in conjunction with the accompanying drawings. The aspects and advantages of the invention will be apparent from and elucidated with reference to the embodiment(s) described hereinafter. Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment.

Where an indefinite or definite article is used when referring to a singular noun e.g. "a" or "an", "the", this includes a plural of that noun unless something else is specifically stated. Where the term "comprising" is used in the present description and claims, it does not exclude other elements or steps. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments, of the invention described herein are capable of operation in other sequences than described or illustrated herein. The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 4th ed., Cold Spring Harbor Press, Plainsview, New York (2012); and Ausubel et al., Current Protocols in Molecular Biology (Supplement 114), John Wiley & Sons, New York (2016), for definitions and terms of the art. The definitions provided herein should not be construed to have a scope less than understood by a person of ordinary skill in the art.

"About" as used herein when referring to a measurable value such as an amount, a temporal duration, and the like, is meant to encompass variations of t 20 % or ± 10 %, more preferably ± 5 %, even more preferably ± 1 %, and still more preferably ± 0.1 % from the specified value, as such variations are appropriate to perform the disclosed methods.

"Orthologues" are genes from different organisms that have originated through speciation, and are also derived from a common ancestral gene.

"Compound" or "test compound" is used herein in the context of a "drug candidate compound" and means any chemical or biological compound, including simple or complex organic and inorganic molecules, peptides, peptido-mimetics, proteins, antibodies, carbohydrates, nucleic acids or derivatives thereof.

The term "subject", "individual" or "patient", used interchangeably herein, relate to any organism for whom therapy or prophylaxis is desired, such as a vertebrate, particularly any mammal, including both a human and another mammal, e.g., an animal such as a rodent, a rabbit, a cow, a sheep, a horse, a dog, a cat, a lama, a pig, or a non-human primate (e.g., a monkey). However, it will be understood that the aforementioned terms do not imply that symptoms are present.

"Synaptic vesicles" or "neurotransmitter vesicles" are defined as small secretory vesicles that contain a neurotransmitter, and are naturally abundantly present organelles of a uniform size inside an axon near the presynaptic membrane. In physiological context, when a nerve impulse moves down the axon of a neuron and arrives at an axon terminal, it stimulates synaptic vesicles in the terminal to discharge neurotransmitters, a mechanism also called "synaptic vesicle trafficking". After fusion with the membrane, a synaptic vesicle releases its contents into the synaptic cleft⁶⁹.

The invention is based on the detailed elucidation of a previously uncharacterized phosphoinositide binding pocket that docks the unconventional TBC protein Skywalker/TBC1D24 to synaptic membranes and is mutated in patients that suffer from diseases such as DOORS syndrome and epilepsy. The direct association of a TBC domain with membrane-phosphoinositides has, not yet been described and we showed that this feature is important for normal Skywalker function. Indeed, flies that express phosphoinositide-binding pocket mutants display presynaptic vesicle trafficking defects and seizure-like behaviour, demonstrating the essential role of phosphoinositide dependent membrane association and vesicle trafficking in TBC1D24-induced neuronal pathology.

Loss of function mutations in human TBC1 D24 lead to several disorders, referred to in the present disclosure as "TBC1 D24-associated disorders", such as severe forms of epilepsy (early onset epilepsy, familial infantile myoclonic epilepsy (FIME), focal epilepsy, dysarthria, myoclonic epilepsy with dystonia, familial malignant migrating partial seizures of infancy, *De Flippo* malignant migrating partial seizures of infancy), variable degrees of intellectual disability, hearing loss (nonsyndromic deafness, autosomal-dominant nonsyndromic hearing loss, and dominant nonsyndromic hearing impairment), and cortical myoclonus and cerebellar ataxia, DOORS syndrome (deafness, onychodystrophy, osteodystrophy, mental retardation and seizures)^{1-14,29}. Although the number of mutations in *TBC1D24* associated with these disorders is rapidly increasing, the molecular mechanisms underlying pathology remain concealed. The surprising effect that Sky cationic pocket mutants harbor lower 4- and 5-phosphorylated phosphoinositide binding affinity raised the opportunity to try rescue the defects associated with the pathological cationic pocket mutants via increasing phosphorylated phosphoinositide levels (e.g. PI(4,5)P₂ and PI(3,4,5)P₃). Several methods can be applied to come to such an effect. In fruit flies, genetic ablation of Synaptojanin, a synaptically enriched phosphoinositide phosphatase, was reduced by 50 % to result in lower mobility of the most severe Sky cationic pocket mutant back to wild type Sky levels and also rescue the neurological defects in the *Sky* mutant flies. The Synaptojanin-1 protein contains a Sac1 phosphatase domain and a 5' phosphatase domain. The latter domain dephosphorylates, among others, PI(4,5)P₂ and PI(3,4,5)P₃. Based on the results obtained in the study leading to this invention, specific inhibitors of phosphoinositide phosphatases, such as the Synaptojanin-1 5' phosphatase domain, are beneficial to revert *TBC1D24*-mutation associated defects. Other ways to obtain such an effect are accomplished by treatment with said inhibitors, leading to increased PI(4,5)P₂ levels in a cell. Additionally, inhibitors affecting both, PI(4,5)P₂ and PI(3,4,5)P₃ levels would then also lead to rescue of TBC1 D24-associated disorders in neuronal cells.

In a first aspect, the invention relates to a phosphoinositide (PI) phosphatase inhibitor to increase phosphatidylinositol 4,5-bisphosphate (PI(4,5)P₂) and/or PI(3,4,5)P₃ levels in a cell, for use in treatment of TBC1D24-associated disorders. The term "inhibitor" refers to any molecule or compound that is capable of blocking or reducing the activity of in this case, a PI phosphatase enzyme. Said inhibitor might be a "small molecule compound", which refers to a low molecular weight (e.g., < 900 Da or < 500 Da) organic compound. The term "treatment" or "treating" or "treat" can be used interchangeably and is defined by a therapeutic intervention that slows, interrupts, arrests, controls, stops, reduces, or reverts the progression or severity of a sign, symptom, disorder, condition, or disease, but does not necessarily involve a total elimination of all disease-related signs, symptoms, conditions, or disorders. A particular embodiment relates to the PI phosphatase inhibitor, for use in treatment of TBC1 D24-associated disorders, wherein said TBC1 D24-associated disorders comprise severe forms of epilepsy (early onset epilepsy, familial infantile myoclonic epilepsy (FIME), focal epilepsy, dysarthria, myoclonic epilepsy with dystonia, familial malignant migrating partial seizures of infancy, *De Flippo* malignant migrating partial seizures of infancy), variable degrees of intellectual disability, hearing loss (nonsyndromic deafness, autosomal-dominant nonsyndromic hearing loss, and dominant nonsyndromic hearing impairment), cortical myoclonus and cerebellar ataxia, and DOORS syndrome (deafness, onychodystrophy, osteodystrophy, mental retardation and seizures). Alternatively, said small molecule compound for use in TBC1D24-associated disorders is a compound for use in severe forms of epilepsy (early onset epilepsy, familial infantile myoclonic epilepsy (FIME), focal epilepsy, dysarthria, myoclonic epilepsy with dystonia, familial malignant migrating partial seizures of infancy, *De Flippo* malignant migrating partial seizures of infancy), variable degrees of intellectual disability, hearing loss (nonsyndromic deafness, autosomal-dominant nonsyndromic hearing loss, and dominant nonsyndromic hearing impairment), cortical myoclonus and cerebellar ataxia, or DOORS syndrome (deafness, onychodystrophy, osteodystrophy, mental retardation and seizures). More particular, said TBC1 D24-associated disorder is refractory epilepsy or DOORS.

Phosphoinositides (Pls) are a group of key signaling and structural lipid molecules involved in a myriad of cellular processes. PI phosphatases, together with PI kinases, are responsible for the conversion of Pls between distinctive phosphorylation states. PI phosphatases are a large collection of enzymes that are evolved from at least two disparate ancestors. For a review on phosphoinositide phosphatase structural insights, see Hsu and Mao (Biochim Biophys Acta. 2015; 1851(6): 698-710). One group of PI phosphatases is distantly related to endonucleases, which applies divalent metal ions for phosphoryl transfer. The other group is related to protein tyrosine phosphatases, which contains a highly conserved active site motif Cys-X5-Arg (CX5R). Based on catalytic mechanisms, PI phosphatases can be categorized into two major classes. One class is the inositol polyphosphate 5-phosphatases, which are Mg²⁺-dependent phosphatases comprising 10 mammalian and 4 yeast members. 5-phosphatases have a characteristic central catalytic domain that exhibits sequence homology to the apurinic/apyrimidic family of endonucleases. Members of this class hydrolyze the D5 phosphate of the inositol ring of both soluble inositol polyphosphates and membrane-bound Pls. The other class is characteristic of a highly conserved CX5R active site motif, of which the cysteine residue functions as the nucleophile while the arginine residue positions the scissile phosphate group. Based on their primary sequences, enzymes in this class can be further divided into four sub-families that include the Sac1 domain containing phosphatase, PTEN, myotubularin, and 4-phosphatase.

The inositol polyphosphate 5-phosphatases are a group of Mg²⁺-dependent phosphatases first characterized in studies on the metabolism of the inositol phosphate second messenger Ins(1 ,4,5)Ps. One example of the 5-phosphatase is synaptojanin 1 (SYNJ1) which was discovered to be responsible for the hydrolysis of PI(4,5)P₂ on synaptic vesicles at the nerve terminal. Besides the 5-phosphatase domain, SYNJ1 and SYNJ2 share an N-terminal Sac1 domain, which was later found to be a member of a new family of PI phosphatases.

So the inhibitors of phosphoinositide phosphatases can be determined in a biochemical assay using a PI phosphatase enzyme and PI substrate.

The terms "blocking", "inhibiting", or "reducing" an enzymatic activity are used interchangeably and mean the observed degree of the catalytic activity is significantly lower in comparison to the catalytic activity observed in the absence of the compound. With significantly lower, it is meant that the activity is reduced preferably, but not by way of limitation, with at least about 5 %, at least about 10 %, at least about 15%, at least about 20%, at least about 25%, at least about 30%, at least about 35%, at least about 40%, at least about 45%, at least about 50%, at least about 60%, at least about 70%, at least about 80%, at least about 90%, at least about 95%, at least about 99%, or at least about 100%.

The terms "stimulating" or "increase", an enzymatic activity or PI(4,5)P₂ levels are used interchangeably and mean the observed degree of the catalytic activity or degree of increased levels is significantly higher in comparison to the catalytic activity or level observed in the absence of the compound. With significantly higher, it is meant that the activity or level is increased preferably, but not by way of limitation, with at least about 5 %, at least about 10 %, at least about 15%, at least about 20%, at least about 25%, at least about 30%, at least about 35%, at least about 40%, at least about 45%, at least about 50%, at least about 60%, at least about 70%, at least about 80%, at least about 90%, at least about 95%, at least about 99%, or at least about 100%.

The specification further provides phosphoinositide phosphatase inhibitors or inhibiting compounds to increase PI(4,5)P₂ levels in a cell, for use in treatment of said TBC1D24-associated disorders, wherein said inhibitors modulate molecules are involved in PI metabolism and are specifically effective in increasing 4- and 5-phosphorylated phosphoinositides, including PI(4,5)P₂ and/or PI(3,4,5)P₃ levels by preferably, but not by way of limitation, at least about 5 %, at least about 10 %, at least about 15%, at least about 20%, at least about 25%, at least about 30%, at least about 35%, at least about 40%, at least about 45%, at least about 50%, at least about 60%, at least about 70%, at least about 80%, or at least about > 90%. Specifically, said increased PI(4,5)P₂ or PI(3,4,5)P₃ levels are detectable by an assay system known by a person skilled in the art, comprising, for instance but not limited to, detection of labeled PI(4,5)P₂ and/or PI(3,4,5)P₃ such as for instance BODIPY-labeled PI(4,5)P₂ and/or PI(3,4,5)P₃ as described⁶¹, or other fluorescent⁶³ or radioactive-labeled assays⁶², or alternative Pi-sensors. Said increased PI(4,5)P₂ levels are in addition detectable by, for instance but not limited to, PI(4,5)P₂-interacting proteins domains (such as PLCd1PH or Tubby) fused to a fluorescent probe such as eGFP ^{66,67} or by using PI(4,5)P₂-specific antibodies⁶⁸.

The specification further provides for PI phosphatase inhibitors to increase PI(4,5)P₂ levels in a cell for use in treatment of said TBC1D24-associated disorders, wherein said inhibition is accomplished herein for any small molecule compound that is capable of blocking or reducing the activity of said PI phosphatase enzymes, i.e. hydrolases belonging to the type II inositol polyphosphate 5-phosphatase and to class EC 3.1.3.36 and catalyzing the dephosphorylation of 1-phosphatidyl-1D-myo-inositol 4,5-bisphosphate (PI(4,5)P₂) resulting in 1-phosphatidyl-1D-myo-inositol 4-phosphate (P(4)IP) and free phosphate. Specifically, said phosphatase activity is for instance detectable by a biochemical assay as known by a person skilled in the art, comprising, but not limited to, measurement of release of phosphate, such as the Malachite Green assay⁶⁰ or PiPer Phosphate Assay Kit (Life Technologies, NY), to measure phosphatase activity. Alternatively, PI phosphatase inhibitors or compounds increasing PI(3,4,5)P₃ levels could also affect TBC1D24/Skywalker, and therefore be considered as therapeutic agents for use in TBC1D24-associated disorders.

It is further envisaged that said inhibitor may, for example and not by way of limitation, inhibit the activity of phosphatidylinositol-3-kinases, stimulate the activity of phosphatidylinositol protein kinase 1 gamma (PIPK1γ), inhibit the activity of phospholipase C (PLC), or increase the activity of phosphatidylinositol-3,4,5-trisphosphate 3-phosphatases such as PTEN to increase PI(4,5)P₂ levels. Without limitation, desired effects of the present disclosure on a treated subject include, in addition to increased PI(4,5)P₂, re-establishing of the TBC1D24 protein mobility via membrane association, and/or a rescue of TBC1 D24-associated disorders/ symptoms.

The specification further provides that said PI phosphatase inhibitor or compound for use in treatment of said TBC1D24-associated disorders, is identified in a biochemical assay, for instance but not limited to, the Malachite Green assay using a PI phosphatase enzyme (e.g. see Example 11).

In another aspect of the invention, the PI phosphatase inhibitor increases the PI(4,5)P₂ levels in a cell via Synaptojanin-1 inhibition. More particular, Synaptojanin-1 is the polyphosphatidylinositide or polyphosphoinositide phosphatase concentrated in presynaptic nerve terminals, where it dephosphorylates a pool of phosphatidylinositol 4,5-bisphosphate implicated in synaptic vesicle recycling. As exemplified, by inhibition of Synaptojanin activity in fruit flies, Sky/TBC1D24-membane interactions were re-established, which provides the molecular insight and consecutive approach to develop viable strategies to bypass the Sky/TBC1D24-induced defects based on Synaptojanin-1 phosphatase inhibition, which involves an increase of PI(4,5)P₂ levels. Moreover, in one embodiment, said increase in PI(4,5)P₂ levels is obtained by Synaptojanin-1 5' phosphatase domain activity inhibition, as said domain enables the removal of a phosphate group from PI(4,5)P₂. The N-terminal Sac1 domain predominantly dephosphorylates PI(3)P and PI(4)P, whereas the 5' phosphatase domain dephosphorylates PI(4,5)P₂ and PI(3,4,5)P₃ at the 5' position of the inositol ring. Hydrolysis of PI(4,5)P₂ is potentially involving both domains in a concerted way, as the 5' phosphatase domain first converts PI(4,5)P₂ to PI(4)P, which is directly channelled to Sac1 catalytic reaction for further dephosphorylation to PI without accumulation of the intermediate. In a particular embodiment accumulation of PI(4,5)P₂ is obtained by inhibition of the 5' phosphatase domain of Synaptojanin-1, for use in treatment of TBC1D24-associated disorders.

On the other hand, like other proteins with a role in endocytosis, Synaptojanin-1 undergoes constitutive phosphorylation mediated by cyclin-dependent kinase 5 (CDK5) in resting synapses and stimulation-dependent dephosphorylation by calcineurin, thereby regulating its function both *in vitro* and in intact synaptosomes. It was shown that Cdk5 phosphorylation inhibited the inositol 5-phosphatase activity of Synaptojanin-1, whereas dephosphorylation by calcineurin stimulated such activity⁶⁴. Therefore the activation of CDK5 by said compounds may be envisaged as well in the regulation of PI(4,5)P₂ turnover at synapses.

The specification further provides that said increase in PI(4,5)P₂ level is obtained with a compound, wherein said compound inhibits phosphatidylinositol-3-kinases, for use in treatment of TBC1D24-associated disorders. Said phosphatidylinositol-3-kinase, is characterized in that this enzyme is a phosphor-transferase belonging to the class I phosphatidylinositol-4,5-bisphosphate 3-kinases (EC 2.7.1.153), converting PI(4,5)P₂ into PI(3,4,5)P₃ using ATP.

The specification further provides for inhibitors to increase PI(4,5)P₂ levels for use in treatment of TBC1D24-associated disorders, wherein said increase is obtained by phospholipase C (PLC) inhibition. PLC cleaves PI(4,5)P₂, which is a phospholipid, into diacyl glycerol (DAG) and inositol 1,4,5-trisphosphate (IP3). Thus PLC has a profound impact on the depletion of PI(4,5)P₂, acting as a membrane anchor, and is critical to the regulation of local PI(4,5)P₂ concentrations within plasma and nuclear membrane.

The specification further provides for said inhibitors to increase said PI(4,5)P₂ levels for use in treatment of TBC1D24-associated disorders, which are characterized in that said PI(4,5)P₂ levels are increased in neuronal cells, wherein neuronal cells or neurons are a type of cell in the central nervous system, which receive, integrate, and pass along information by releasing neurotransmitters. Related to the increase of PI(4,5)P₂ levels, compounds or PI phosphatase inhibitors leading to increased PI(3,4,5)P₃ levels in neuronal cells would be considered for use in treatment of TBC1D24-associated disorders as well. Neurotransmitters are chemicals that cross-over from the terminal button at the end of an axon over the synapse to the neighbouring neuron. Non-limiting examples of neuron cells are primary cortical neurons, primary basal forebrain cholinergic neurons, primary neural stem cells, sensory neurons (e.g. retinal cells, olfactory epithelium cells), motoneurons (e.g. spinal motor neurons, pyramidal neurons, Purkinje cells) and interneurons (e.g. dorsal root ganglia cells).

Another embodiment of the invention relates to a PI phosphatase inhibitor, for use in treatment of TBC1D24-associated disorders, characterized in that said inhibitor is selected from a list consisting of bpV(pic), perifosine, edelfosine, miltefosine, erufosine, or YU144118, or a derivative thereof, wherein a derivative means any compound produced from one of said listed original compounds either directly or by modification or partial substitution of the original compound core, by a chemical reaction, resulting in a salt, or a hydrate form.

In one embodiment of the present invention, the PI phosphatase inhibitor, for use in treatment of TBC1D24-associated disorders, is an alkyl phospholipid. Synthetic Alkyl phospholipids or ether lipids are lipids in which one or more of the carbon atoms on glycerol is bound to an alkyl chain via an ether linkage, as opposed to the usual ester linkage. Several clinically relevant alkyl phospholipid compounds have been developed (e.g. Figure 11), all being derived from the natural compound lysophosphatidylcholine. Alkyl phospholipids have been described to play a role in as anticancer agents (e.g. Van Blitterswijk and Verheij, 2013. Biochim. & Biophys. Acta, 1831:663-674; Jaffrès et al., 2016. Pharmacol Ther. 165:114-31), and to inhibit PI phosphatase activity.

One embodiment provides the alkyl phospholipid compound inhibitor, for use in treatment of TBC1D24-associated disorders, wherein said alkyl phospholipid is miltefosine, or a derivative thereof. In particular, the invention provides the use of miltefosine or a derivative thereof, at a concentration that increases PI(4,5)P₂ for use in treatment of TBC1D24-associated disorders, with potential for a novel therapeutic application for said drug compound, also launched as the first approved drug for cutaneous or mucosal leishmaniasis via oral administration as Impavido (and Miltex), owned by Zentaris GmbH (Aeterna), and launched as well as a breast cancer therapy by ASTA Medica Oncology (now Baxter Oncology). Another embodiment relates to the alkyl phospholipid compound inhibitor for use in treatment of TBC1D24-associated disorders, wherein said compound is perifosine, or a derivative thereof. Perifosine may be administered to achieve a local concentration in the area of cells to be treated orto increase local PI(4,5)P₂ through inhibition of PLC, or through inhibition of PI phosphatase activity, and preferably resulting in decrease in TBC1D24-associated disorder symptoms. Alternatively, the alkyl phospholipid compound for use in treatment of TBC1D24-associated disorders, is edelfosine, or a derivative thereof. Or the alkyl phospholipid compound, for use in treatment of TBC1D24-associated disorders, is erufosine, or a derivative thereof, a compound known to allow BBB crossing. Furthermore, in another embodiment, the invention provides for the use of bpV(pic) or a derivative thereof, known in the art to block phosphatidylinositide phosphatase activity, thereby in a preferred embodiment increasing PI(4,5)P₂ levels for use in treatment of diseases concerning pathological *TBC1D24* mutations. Another embodiment of the present invention provides YU144118 or a derivative thereof as a compound selected from the list of compounds for use in treatment of TBC1D24-associated disorders, wherein YU144118 is known to inhibit phosphatidylinositide phosphatase activity⁶⁵, revealing a potent inhibitor leading to increase in PI(4,5)P₂ in a cell.

The specification further provides that said PI phosphatase inhibitors are analysed in flies for behavioural rescue phenotypes, such as flight and seizure assay (see Examples). It is further envisaged that further confirmation of these observations is done by feeding *Sky*^{*1*/*2*} mutants expressing Sky^{R79C} with PI(4,5)P₂ increasing compounds, resulting in normal GFP Sky^{R79C} mobility in the FRAP assay, indicating that increasing the PI(4,5)P₂ levels restricts the diffusion of the GFP-Sky^{R79C} mutant at synapses. Furthermore, while expression of the Sky^{R79C} mutant in *sky*^{*1*/*2*} flies could only partially reduce the number of FM 1-43 accumulations, PI(4,5)Pzstimulating compound feeding rescues the FM 1-43 accumulation defect, and no significant difference from controls (*yw*; *FRT40A*) or *sky*^{*1*/*2*} mutants expressing wild type Sky^{WT} would be observed.

As described herein but outside the subject-matter of the claims, human embryonic stem cells (H9) are mutated using CRISPR/CAS9 technology in the TBC1D24 positive pocket encoding residue. The mutated cells are allowed to differentiate further into glutamatergic cortical neurons, followed by transfection with a lentivirus expressing synaptophluorin. Synaptophluorin allows measurement or synaptic vesicle fusion rates, which is a disrupted parameter in fly *sky* mutants. A second measurement of the synaptic vesicle fusion rate when stimulating the neurons at high frequency (field stimulation) in the absence and presence of PI(4,5)P₂ stimulating compounds further allows to identify the impact of each compound or PI phosphatase inhibitor tested.

In a second aspect outside the subject-matter of the claims, a method for producing a PI phosphatase inhibitor compound is provided, characterized in that said inhibitor is a 5' phosphatase domain-specific Synaptojanin-1 inhibitor, wherein said method comprises the steps of: a) providing an *in vitro* system comprising the Synaptojanin-1 5' phosphatase domain protein as depicted in SEQ ID NO:1 or a homologue with at least 95 % amino acid identity thereof and PI(4,5)P₂ or PI(3,4,5)P₃ substrates, or derivatives thereof; b) administering a test compound to said *in vitro* system; c) monitoring phosphatase activity in said *in vitro* test system, wherein, a reduction in said phosphatase activity as compared to phosphatase activity measured under the same test conditions in the same system without the test compound, identifies said test compound as a Synaptojanin-1 inhibitor, more particular a 5' phosphatase-specific Synaptojanin-1 inhibitor. Said method provides inhibitors or compounds for use in treatment of TBC1 D24-associated disorders.

Said *"in vitro* system" refers to a system comprising at least the necessary components and environment to execute said method, and makes use of biological molecules, organisms, a cell (or part of a cell) outside of their normal naturally-occurring environment, permitting a more detailed, more convenient, or more efficient analysis than can be done with whole organisms. Non-limiting examples of said *in vitro* system as described herein are neuronal cells, induced pluripotent stem cells (also known as iPS cells or iPSCs), or a buffer condition suitable to perform said monitoring assay. The substrates provided in said *in vitro* system, PI(4,5)P₂ or PI(3,4,5)P₃, or derivatives thereof include labelled, tagged or any other form of PI(4,5)P₂ or PI(3,4,5)P₃ derivatives that provide an advantage to the assay, e.g. in detecting, quantifying, or visualizing the substrates.

The specification further envisages said method provided in step a) by said *in vitro* system also contains the Synaptojanin-1 5' phosphatase domain protein as depicted in SEQ ID NO:1 or a homologue with at least 95 % amino acid identity thereof. The specification further envisages said method provided in step a) by said *in vitro* system contains Synaptojanin-1 Sac1-domain inactivated mutant protein as depicted in SEQ ID NO:2 or a homologue with at least 95 % amino acid identity thereof, instead of the Synaptojanin-1 5' phosphatase domain protein as depicted in SEQ ID NO:1 or a homologue with at least 95 % amino acid identity thereof.

"Homologue" or "Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived. According to the present invention, the degree of identity between a given reference amino acid sequence and an amino acid sequence which is a variant of said given amino acid sequence will preferably be at least about 95%, 96%, 97%, 98%, or 99%. The degree of identity is given preferably for an amino acid region which is at least about 90% or about 100% of the entire length of the reference amino acid sequence. For example, if the reference amino acid sequence consists of 200 amino acids, the degree of identity is given preferably for at least about 180, or about 200 amino acids, preferably continuous amino acids. In preferred embodiments, the degree/percentage of identity is given for the entire length of the reference amino acid sequence. The term "amino acid identity" as used herein refers to the extent that sequences are identical on an amino acid-by-amino acid basis over a window of comparison. Thus, a "percentage of sequence identity" is calculated by comparing two optimally aligned sequences over the window of comparison, determining the number of positions at which the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. The alignment for determining sequence identity, can be done with art known tools, preferably using the best sequence alignment, for example, using CLC main Workbench (CLC bio) or Align, using standard settings, preferably EMBOSS::needle, Matrix: Blosum62, Gap Open 10.0, Gap Extend 0.5.

Accordingly, the invention relates to the use of SEQ ID NO: 1-defined Synaptojanin-1 5' phosphatase domain protein sequence, and/or the use of SEQ ID NO: 2-defined Synaptojanin-1 Sac1-domain inactivated mutant protein sequence, thereby providing a suitable system for the test compounds to identify 5' phosphatase domain-specific Synaptojanin-1 inhibitors.
SEQ ID NO: 1 depicts the amino acid sequence of the 5' phosphatase domain of Synaptojanin-1 (346 aa).
**SEQ ID NO: 1:** 5' phosphatase domain of *human* Synaptojanin-1 protein sequence (346 aa): 
SKPKKIRVCVGTWNVNGGKQFRSIAFKNQTLTDWLLDAPKLAGIQEFQDKRSKPTDIFAIGFEEMVELNAGNIVSAST
TNQKLWAVELQKTISRDNKYVLLASEQLVGVCLFVFIRPQHAPFIRDVAVDTVKTGMGGATGNKGAVAIRMLFHTTS
LCFVCSHFAAGQSQVKERNEDFIEIARKLSFPMGRMLFSHDYVFWCGDFNYRIDLPNEEVKELIRQQNWDSLIAGDQ
LINQKNAGQVFRGFLEGKVTFAPTYKYDLFSDDYDTSEKCRTPAWTDRVLWRRRKWPFDRSAEDLDLLNASFQDESKI
LYTWTPGTLLHYGRAELKTSDHRPVVALIDIDIFEV
SEQ ID NO: 2 depicts the amino acid sequence of the *human* Synaptojanin-1 Sac1-domain inactivated mutant (C383S; 1311 aa).
**SEQ ID NO: 2:** *Human* Synaptojanin-1 Sac1-domain inactivated mutant protein sequence (C383S; 1311 aa): 
MAFSKGFRIYHKLDPPPFSLIVETRHKEECLMFESGAVAVLSSAEKEAIKGTYSKVLDAYGLLGVLRLNLGDTMLHYLVL
VTGCMSVGKIQESEVFRVTSTEFISLRIDSSDEDRISEVRKVLNSGNFYFAWSASGISLDLSLNAHRSMQEQTTDNRFF
WNQSLHLHLKHYGVNCDDWLLRLMCGGVEIRTIYAAHKQAKACLISRLSCERAGTRFNVRGTNDDGHVANFVETEQ
VVYLDDSVSSFIQIRGSVPLFWEQPGLQVGSHRVRMSRGFEANAPAFDRHFRTLKNLYGRQIIVNLLGSKEGEHMLSK
AFQSHLKASEHAADIQMVNFDYHQMVKGGKAEKLHSVLKPQVQKFLDYGFFYFNGSEVQRCQSGTVRTNSLDCLDR
TNSVQAFLGLEMLAKQLEALGLAEKPQLVTRFQEVFRSMWSVNGDSISKIYAGTGALEGKAKLKDGARSVTRTIQNNF
FDSSKQEAIDVLLLGNTLNSDLADKARALLTTGSLRVSEQTLQSASSKVLKSMCENFYKYSKPKKIRVCVGTWNVNGGK
QFRSIAFKNQTLTDWLLDAPKLAGIQEFQDKRSKPTDIFAIGFEEMVELNAGNIVSASTTNQKLWAVELQKTISRDNKY
VLLASEQLVGVCLFVFIRPQHAPFIRDVAVDTVKTGMGGATGNKGAVAIRMLFHTTSLCFVCSHFAAGQSQVKERNE
 
DFIEIARKLSFPMGRMLFSHDYVFWCGDFNYRIDLPNEEVKELIRQQNWDSLIAGDQLINQKNAGQVFRGFLEGKVTF
APTYKYDLFSDDYDTSEKCRTPAWTDRVLWRRRKWPFDRSAEDLDLLNASFQDESKILYTWTPGTLLHYGRAELKTSD
HRPVVALIDIDIFEVEAEERQNIYKEVIAVQGPPDGTVLVSIKSSLPENNFFDDALIDELLQQFASFGEVILIRFVEDKMW
VTFLEGSSALNVLSLNGKELLNRTITIALKSPDWIKNLEEEMSLEKISIALPSSTSSTLLGEDAEVAADFDMEGDVDDYSA
EVEELLPQHLQPSSSSGLGTSPSSSPRTSPCQSPTISEGPVPSLPIRPSRAPSRTPGPPSAQSSPIDAQPATPLPQKDPAQ
PLEPKRPPPPRPVAPPTRPAPPQRPPPPSGARSPAPTRKEFGGIGAPPSPGVARREMEAPKSPGTTRKDNIGRSQPSP
QAGLAGPGPAGYSTARPTIPPRAGVISAPQSHARASAGRLTPESQSKTSETSKGSTFLPEPLKPQAAFPPQSSLPPPAQ
RLQEPLVPVAAPMPQSGPQPNLETPPQPPPRSRSSHSLPSEASSQPQQEQPSG
Derived from:
   > gi|270232**1**|gb|AAC51921.1| synaptojanin [*Homo sapiens*]

The specification further envisages that, monitoring of said PI phosphatase activity in said method for producing said 5' phosphatase domain-specific Synaptojanin-1 inhibitors, is characterized in that release of phosphate is detected. Such a measurement can be done in different embodiments outside the subject-matter of the claims in several ways⁶⁵, for example, but non-limiting, via the malachite green assay⁶⁰ or PiPer Phosphate Assay Kit (Life Technologies, NY), or detection of labeled PI(4,5)P₂ such as for instance BODIPY-labeled PI(4,5)P₂ as described⁶¹, or other fluorescent⁶³ or radioactive-labeled assays⁶², or using alternative Pi-sensors.

In other embodiments outside the subject-matter of the claims, monitoring of said PI phosphatase activity are performed by, for instance but not limited to, PI(4,5)P₂-interacting proteins domains (such as PLCd1PH or Tubby) fused to a fluorescent probe such as eGFP ^{66,67} or by using PI(4,5)P₂-specific antibodies⁶⁸. Said monitoring will result in identification of a hit compound when reduced phosphatase activity is observed as compared to a sample without test compound, wherein said reduction is preferably, but not by way of limitation, at least about 5 %, at least about 10 %, at least about 15%, at least about 20%, at least about 25%, at least about 30%, at least about 35%, at least about 40%, at least about 45%, at least about 50%, at least about 60%, at least about 70%, at least about 80%, at least about 90%, at least about 95%, at least about 99%, or at least about 100%.

The following examples are provided to better illustrate particular embodiments, and they should not be considered limiting the application. The application is limited only by the claims.

### EXAMPLES

### 1. The crystal structure of the Skywalker TBC domain reveals an unanticipated cationic pocket

To determine the crystal structure of the TBC domain of Skywalker (Sky1-353) the protein was expressed in E. *coli* and purified to homogeneity. X-ray diffraction data for crystals of the native protein were collected at 2.5 Å resolution (**Table 1**). Given the relatively low sequence identity of Sky1-353 compared to other TBC domains for which the structure is available, the structure was solved via single-wavelength anomalous diffraction (SAD) on a selenomethionine-derivative. The asymmetric unit of the crystal contains one copy of Sky1-353, and clear electron density is present for most of the peptide chain and side chains. Only the N-terminal regions spanning residues 1-26 and 37-54 as well as the last residue (353) are not modelled due to lack of electron density. Sky1-353 superimposes well with the eleven known TBC domain-containing proteins in the PDB (rmsd values in the range of 2.3 to 3.3 Å2 over ~230 aligned residues). Similar to other TBC domains, Sky1-353 is all-helical and can be divided into an N-terminal and a C-terminal lobe²⁷ (**Fig. 1a**). Superposition of Sky1-353 with the TBC domain of Gyp1 (PDB 2G77) indicates that the main differences in conformation occur at the interface of the N- and C terminal lobes where small GTPases are expected to bind^{18,19} (**Fig. 1b**). The most notable difference with the TBC domain of Gyp1 concerns helix α5 of Gyp1, which is replaced by a loop (residues 120 to 148) in Sky1-353, causing also a different conformation of the loop connecting α6 to α7 (using the Gyp1 numbering of secondary structure elements). Precisely these two regions in Gyp1 comprise the R and Q fingers, important for GAP activity in most TBC proteins. The residues aligning with these R and Q residues in the Sky sequence, H140 and P172, respectively, adopt different spatial positions in the structure (**Fig. 1b**). Hence, we provide structural evidence that the typical R and Q fingers are absent in Sky, implicating that the Sky-GAP cannot function via a mechanism similar to that used by 'classical' Rab-GAP proteins.

To identify additional unique features of the Sky TBC domain we calculated the conservation scores of the amino acid residues with the Consurf algorithm and using an alignment of 250 Sky homologs containing a TBC and a TLDc domain²⁸. The most striking feature is a region of high conservation at the interface of the N-terminal and C-terminal lobe on the opposite side of the GTPase binding surface (**Fig. 1c**). This very conserved region in Skywalker corresponds to a highly positively charged pocket (**Fig. 1d**) that is lined by residues from the second helix of the N-terminal lobe (residues 71 to 80), the connecting loop between the N- and C-terminal lobe (residues 199 to 210), a part of the fifth helix of the C-terminal lobe (residues 277 to 281) and the two last C-terminal helices (residues 324 to 352). The cationic character of this pocket is caused by the presence of 4 lysine and 4 arginine residues: K71, R72, K75, R79, K203, K277, R281and R335. The positive pocket also contains a sulfate ion that we added during protein purification and crystallization to increase the thermostability of the protein. This sulfate ion is surrounded by and potentially interacts with the positive charges of K75, R79 and R281. The residues in this positive pocket are all evolutionary well conserved across species and they are unique to the Skywalker/TBC1 D24-family, since a similar positive pocket is not present in any of the other eleven TBC structures available in the protein database.

**Table 1: Data collection and refinement statistics**

| | **Sky1-353 SeMet** | **Sky1-353** | **Sky1-353-IP₃** |
|---|---|---|---|
| **Data statistics** | | | |
| X-ray source | SOLEIL Proxima 2 | SOLElL Proxima 2 | ESRF ID23-1 |
| Wavelength (Å) | 0.9791 | 0.9801 | 0.9762 |
| Space group | P4₁ 2₁ 2 | P4₁ 2₁ 2 | P4₂ 2₁ 2 |
| Unit Cell parameters (Å. °) | a = b = 87.3 c = 187.7 | a = 6 = 97.3 c = 187.4 | a = b = 87.7 c = 97.1 |
| | α = β = γ = 90 | a = β = γ = 90 | α = β = γ = 90 |
| Resolution range (Å) | 43.94-3.28 (3.54-3.28) | 43.89-2.50 (2.65-2.50) | 39.23-2.30 (2.44-2.30) |
| Observed reflections | 208426 (39867) | 203106 (32356) | 217258 (33760) |
| Unique reflections | 11679 (2258) | 25773 (4042) | 17353 (2656) |
| Completeness (%) | 99.1 (95.7) | 99.8 (99.5) | 99.4 (95.9) |
| Rmerge (I) | 0.266 (0.717) | 0.102 (1.035) | 0.095 (0.802) |
| Redundancy | 17.8 (17.7) | 7.9 (8.0) | 12.5 (12.7) |
| Mean *I*/σ(*I*) | 11.5 (5.2) | 17.06 (2.07) | 18.18 (2.43) |

| **Refinement statistics** | | | |
|---|---|---|---|
| Rwork/Rfree^{a} | | 0.218/0.265 | 0.230/0.257 |
| No of protein atoms | | 2510 | 2308 |
| No of water | | 47 | 30 |
| Ion/Ligand | | SO₄²⁻ x 1 | IP₃ x 1 |
| B factor (Å²) | | 50.3 | 67.0 |
| Rmsd bond (Å) | | 0.003 | 0.004 |
| Rmsd angle (°) | | 0.699 | 0.806 |
| Ramachanran favored/outlier (%) | | 96/0.3 | 95/0.4 |

Values for the highest-resolution shell are given in parentheses. ^{a} 5% of the reflections are used for the Rfree calculation.

### 2. DOORS-associated mutations in TBC1D24 map to the cationic pocket

Mutations in TBC1D24 cause epilepsy, DOORS syndrome and non-syndromic deafness. To gain insight into the mechanisms of pathology we mapped the pathogenic mutations14 on the structure. 19 of the 24 different known mutations are missense mutations and most of them are located in the TBC domain of TBC1D24. Sequence alignment of TBC1D24 and Sky allows us to map 14 of these 19 residues on the structure (**Fig. 2**). A first class of affected residues are buried in the protein core and mutations may thus decrease conformational stability (e.g. G150, M268 and F290, using the Sky numbering). Eleven residues are (partially) surface exposed. Interestingly, only four of these surface-exposed residues map to the 'GTPase interaction surface': H145, W253, K306 and G310 (corresponding to C105, R214, K266 and R270 in TBC1D24) (**Fig. 2b**)**.** Four other surface exposed residues, D109, E187, C193 and V215 (corresponding to D70, D147, E153 and S178 in TBC1D24), are dispersed on the surface and may be involved in interaction with the TLDc domain, the linker region or with other potential binding partners. The majority (70%) of DOORS syndrome patients with lesions in *TBC1D24,* carry mutations in either one of two arginine residues, R40 and R242¹³. These residues cluster together in the crystal structure (R79 and R281, using Sky numbering) and are central to the positively charged pocket in the Sky1-353 structure. Moreover, a third R residue that has been found mutated in non-syndromic deafness patients (R293 in TBC1D24 - R335 in Skywalker) also maps to the same positively charged pocket29. These observations further underpin the importance of the cationic pocket and its significance in TBC1D24-related disease.

### 3. The cationic pocket in Skywalker is a previously uncharacterized phosphoinositide-binding module

TBC1 D24 and Skywalker regulate vesicle trafficking, a process that requires membrane interactions^{20,24,30}. We therefore hypothesized that the positively charged pocket of Sky serves as a binding module to negatively charged membranes. We used a liposome flotation assay and mixed Sky1-353 with liposomes produced from Fetch-extracted total brain lipids and centrifuged the sample in a sucrose gradient. After recovering six fractions from the top to the bottom of the centrifugation tube we find that Sky1-353 is present in the top fractions that contain the liposomes. By contrast, Sky1-353 is largely absent from the bottom fractions that do not contain liposomes (**Fig. 3a**). Hence, the TBC domain of Sky can associate with liposomes. A major class of lipids that regulates presynaptic activity are phosphoinositides³¹. Lipid strip interaction experiments using Sky1-353 are indeed compatible with Sky interacting with phosphoinositides. We therefore resorted to liposome flotation assays using liposomes composed of phosphatidylcholine and phosphatidylserine and supplemented with different types of phosphoinositides (**Fig. 3b**). Sky1-353 does not bind to liposomes devoid of phosphoinositides or containing non-phosphorylated phosphatidylinositol (PI) (**Fig. 3b**). Similarly, Sky1-353 does not bind to liposomes with PI(3)P. We do observe weak but significant binding of Sky1-353 to liposomes with phosphoinositides containing a phosphate on position 4 or 5 (PI(4)P and PI(5)P), and the strongest binding is observed to liposomes with phosphoinositides phosphorylated on both position 4 and 5 (PI(4,5)P₂ and PI(3,4,5)P₃). Varying the concentration of PI(4,5)P₂ from 0% to 5%, shows that binding of Sky1-353 is observed from the lowest concentration tested (0.5%), while full binding is reached at 2% PI(4,5)P₂. Moreover, the binding can be reversed by adding increasing concentrations of salt, as would be expected from binding that is mainly driven by electrostatic interactions. Hence, Sky electrostatically interacts with phosphoinositides phosphorylated in the 4 and 5 position, and most strongly to PI(4,5)P₂ and PI(3,4,5)P₃, both implicated in synaptic vesicle trafficking^{32,33}.

To obtain a quantitative estimate of the affinity of Sky1-353 for PI(4,5)P₂, we determined the thermal stability (melting temperature, Tm) of Sky1-353 in the presence of increasing amounts of inositol 1,4,5-trisphosphate (IP3), the soluble head group of PI(4,5)P₂, using thermal shift assays³⁴. To ensure the positive pocket in Sky was devoid of any ligand prior to performing this experiment we extensively dialyzed the protein against sulfate ion-free buffer. A saturating (2 mM) concentration of IP3 increases the Tm of Sky1-353 by about 13°C. Plotting the Tm value versus the IP3 concentration yields a binding curve that can be fitted to a quadratic equation yielding a binding dissociation constant (KD) of 19 ± 3 µM (Fig. 3d). This KD value for IP3 is similar to the binding dissociation constants that have been reported for other protein domains that bind phosphoinositides^{35,36}. In comparison, an approximately 1000-fold lower affinity for 187 sulfate (KD = 22 ± 3 mM) is obtained using the same approach. Taken together, our data suggest that Sky1-353 shows high affinity for the PI(4,5)P₂ head group.

To provide molecular insight into the mode of phosphoinositide binding by Sky we determined the crystal structure of Sky1-353 in complex with IP3. We solved the structure of this complex by molecular replacement using the Sky1-353 structure as a model and refined it to a resolution of 2.30 Å (Table 1). No major conformational changes occur between the structures of Sky1-353 (PDB 5HJN) and Sky1-353-IP3 (PDB 5HJQ). The structure shows very clearly defined electron density for the IP3 molecule in the positively charged pocket of the TBC domain and we were able to unambiguously assign the phosphates to positions 1, 4 and 5 of the inositol ring (**Fig. 3e**). The IP3 molecule is bound via multiple interactions with residues K75, R79, K277, R281, R335, G336 and T339. The phosphate group at position 1 that in a phosphoinositide molecule links the inositol group to the diacylglycerol chain, makes an ionic interaction with the side chain of R335 and forms a bifurcated hydrogen bond with the main chain amino group of G336. The phosphate group on position 4 makes an ionic interaction with K277 and forms hydrogen bonds with the side chain and main chain amino group of T339. The phosphate group on position 5 is the most tightly chelated as it interacts with K75, R79 (bifurcated), K277 and R281 (bifurcated). Finally, the inositol hydroxyl group at position 6 might interact with R281 and R335. The hydroxyl groups on position 2 and 3 are pointing towards the outside of the binding pocket and do not interact with the protein. These structural observations explain the specificity of Sky for phosphatidylinositol phosphorylated at position 4 and/or 5, with the 5-position seemingly being the strongest determinant for binding and possibly recognition. The data also indicate that Sky contains a unique phosphoinositide binding pocket that is distinct from known phosphoinositide binding domains.

### 4. Analogs to pathogenic mutations lower phosphoinositide binding affinity in Sky

To determine if pathogenic mutations in the positive pocket of TBC1D24 affect phosphoinositide binding we assessed the binding of mutant Sky1-353 proteins to liposomes containing PI(4,5)P₂. We created and purified the R79C, R281C and R335P mutants that correspond to the R40C, R242C and R293P mutations in *TBC1D24* patients (**Fig. 2a**). Additionally, we also created a Sky1-353 mutant in which these 3 arginine residues are simultaneously replaced by negatively charged glutamate residues (3GIu). Although the latter mutant could not be purified to homogeneity, due to low expression levels in *E*. *coli,* the amount of obtained protein was sufficient for conducting the liposome flotation assays. Finally, flotation was tested under less (2% PI(4,5)P₂) and more (0.5% PI(4,5)P₂) stringent conditions. Both the R79C and 3GIu mutation completely disrupt the interaction with PI(4,5)P₂ (**Fig. 3c**). For the R281C and R335P mutants a milder effect on PI(4,5)P₂ binding is observed. At 2% PI(4,5)P₂ the R281C mutant is still mainly found in the top fractions, although a small amount is observed in the bottom fraction. However, at 0.5 % PI(4,5)P₂, the interaction of this mutant with the liposomes is nearly completely lost. Finally, the R335P mutant is retrieved entirely in the top fraction under the experimental conditions (see below).

Subsequently, the effect of the pathogenic point mutations was further quantified by measuring the affinity for IP3 using thermal shift assays. The thermal stability of the R79C and R281C mutants in absence of ligands (Tm = 32°C) is somewhat lower compared to the wild type protein (Tm = 34°C) and the R335P mutant (Tm = 35°C). While the presence of a saturating amount of IP3 causes a thermal stability shift of 13°C in wild type Sky1-353, the R79C and R281C mutants show only a small increase in thermal stability by 4°C and the R335P mutant by 8°C. Fitting of the titration curves yields a Kd of 325 ± 200 µM for R79C, 180 ± 100 µM for R281C and 67 ± 17 µM for R335P, compared to a Kd of 19 ± 3 µM for the wild type protein (**Fig. 3d**). Hence, the affinity of the mutant proteins for IP3 is significantly decreased by 17 to 3.5 fold compared to the wild type protein. The observed effect of the clinical mutations on the affinity for IP3 is in agreement with the liposome flotation assays that showed the largest effect for the R79C mutant, with an intermediate effect for the R281C mutant and the smallest effect for R335P. These experimental data moreover correspond with our structural data where R79 and R281 both form bifurcated electrostatic interactions with the phosphate on position 5, while R335 forms a single electrostatic interaction with the phosphate group on position 1 (**Fig. 3e**). These data thus present a link between pathogenic mutations and the phosphoinositide-binding function of Sky/TBC1D24.

### 5. The cationic pocket restricts Sky mobility at synapses in vivo

Sky is prominently present at synapses where it regulates the trafficking of synaptic vesicles²⁰, a process intimately connected to vesicle's phosphoinositide composition³². In this respect, we reasoned that binding to phosphoinositides in the membrane could be critical for Sky to exert its function and we hypothesized that *in vivo,* the ability of Sky to bind to membranes would restrict its diffusion. To test Sky mobility at synapses we turned to *Drosophila* neuromuscular junctions (NMJs) and assessed the recovery of GFP fluorescence after photobleaching of wild type and mutant GFP Sky (FRAP) (**Fig. 4a****,b**). We created transgenic fruit flies that express N-terminal GFP fusion constructs of Sky containing the mutations that showed the strongest effects on PI(4,5)P₂ and IP3 binding affinity (GFP-SkyR79C, GFP-SkyR281C, GFP-Sky3Glu). All UAS constructs as well as wild type GFP-Sky (GFP-SkyWT) were inserted in the same genomic locus and proteins were expressed using a neuron-specific driver (nSybGal4) in *sky1*/*2* mutants *(sky1*/*2* is a severe hypomorphic *sky* mutant²⁰). Assessment of GFP fluorescence levels in the ventral nerve cord and at NMJ boutons indicates that the mutant Sky proteins are (1) all present at synapse-rich areas in the neuropile of the ventral nerve cord and at synaptic boutons and (2) expressed at a level at least as high as that observed for GFP-SkyWT. While the Sky3Glu mutant was difficult to express in *E coli,* when expressed in flies, we do not observe a difference in localization or expression level compared to the other GFP-Sky mutants that we expressed. These data indicate that the mutant GFP-Sky proteins are stable *in vivo* and they are trafficked properly to synapses. When we next assessed GFP-Sky mobility using FRAP, we find that the GFP-Sky mutants are significantly more mobile when compared to wild type GFP-Sky. Indeed, the fluorescence in synaptic boutons expressing GFP-SkyR79C, GFP-SkyR281C as well as GFP-Sky3Glu recovers fasterthan in boutons expressing wild type GFP-Sky (**Fig. 4b**). These data suggest that Sky-membrane interactions via its cationic pocket restrict Sky mobility at synapses.

### 6. The cationic pocket is critical for Sky function

PI(4,5)P₂ and PI(3,4,5)P₃ are present in (synaptic) membranes and are also critical during synaptic endocytosis^{33,37}. Given that Sky regulates the trafficking of vesicles we wondered whether membrane binding is functionally relevant *in vivo.* We expressed wild type and mutant Sky in neurons (nSybGal4) of *sky1*/*2* mutants and assessed vesicle trafficking. We stimulated the neurons in the presence of FM 1-43, a fluorescent dye that is internalized into newly endocytosed vesicles, and then imaged synaptic fluorescence. In *sky1*/*2* mutants, FM 1-43 accumulates in sub boutonic membrane structures that previously²⁰ were shown to harbor markers of sorting endosomes (FYVE-GFP, Rab5) (**Fig. 5a****,b**). This defect is rescued by expressing wild type Sky.

In contrast, expression of the Sky point mutants (R79C, R281C and 3Glu) only partly rescues the defect in *sky1*/*2* mutants, and several FM 1-43 accumulations are still present. Quantification of the data indicates that the conditions where we express mutant Sky in *sky1*/*2* mutants are different from both controls (yw; *FRT40A* or *sky1*/*2* expressing SkyWT: p<0.0001) and from *sky1*/*2* mutants (p<0.0001) (**Fig. 5a****,b**), with one exception, SkyR281C where the *p* value with SkyWT is 0.0728. Further scrutinizing this defect, we also performed electron microscopy of stimulated (60 mM KCI) presynaptic terminals. As shown in **Fig. 5c,d****,** *sky1*/*2* mutants expressing wild type Sky do not show an accumulation of large cisternal/endosomal-like structures and synaptic boutons are filled with normal sized synaptic vesicles, very similar to wild type control boutons (yw; *FRT40A*). In contrast *sky1*/*2* mutants harbor many cisternal/endosomal-like profiles, consistent with the FM 1-43 that also appears in clumps in these animals. When mutant SkyR79C or Sky3Glu is expressed in *sky1*/*2* mutants, the number of cisternal/endosomal-like profiles at synaptic boutons is significantly lower than in *sky1*/*2* mutants, but still significantly higher than when expressing wild type Sky (p=0.0005) (**Fig.5d**)**.** Hence, in accordance with the FM 1-43 results, the EM data indicate that the cationic phosphoinositide-binding pocket is required for normal Sky activity at synaptic terminals, preventing the accumulation of excessive endosomal compartments upon stimulation.

### 7. Pathogenic cationic pocket mutations cause neurological and epilepsy-like defects in fruit flies

*Sky*^{*1*/*2*} mutant flies expressing cationic pocket mutants display synaptic vesicle trafficking defects and patients containing the corresponding mutations suffer from epilepsy and other neuronal defects. We therefore assessed if the cationic pocket-mutant flies show behavioural defects as well. *Sky* null and severe hypomorphic mutants die during development, but expression of wild type Sky only in the nervous system rescues them to adulthood (**Fig. 6a**). Likewise, *sky*^{*1*/*2*} mutants expressing positive-pocket-mutant Sky also survive. However, *sky* mutant flies expressing Sky^{3Glu} or Sky^{R79C} display frequent and severe loss of coordination and most of the animals do not fly (**Fig. 6a****-c**). Furthermore these flies undergo epileptic-like seizures when aroused (**Fig. 6a,b****,** see methods), highly reminiscent of other fly seizure models^{38,39}.

Temperature alters the strength of synaptic transmission in fruit flies and several synaptic transmission mutants and mutants that undergo seizures are also sensitive to changes in temperature. We find that *sky*^{*1*/*2*} mutants expressing Sky^{3Glu} or Sky^{R79C} lose coordination and some flies fail to stand when incubated at temperatures above 34°C for more than 3 min (**Fig. 6d**). In contrast, control flies or *sky* mutants expressing wild type Sky in the nervous system still stand after 3 min at 38°C. Furthermore, when the flies are placed at 36°C, *sky*^{*1*/*2*} mutants expressing Sky^{3Glu} or Sky^{R79C} drop, while the majority of *sky*^{*1*/*2*} mutants expressing wild type Sky still stand after 80 min (**Fig. 6e**). Note however that *sky1*/*2* mutants expressing Sky^{3Glu} drop significantly faster than those expressing Sky^{R79C}. Hence, perturbation of the positive pocket in Sky causes increased sensitivity to temperature. The severity of this sensitivity correlates with the severity of the perturbation in the positive pocket (Sky^{3Glu} > Sky^{R79C} > Sky^{WT} = control).

Finally, to assess the behaviour of *sky* mutants that express Sky^{3Glu} or Sky^{R79C} under basal conditions we measured the activity of individual flies over a 24 h period (12 h light and 12 h dark). Activity is monitored in an automated fashion by assessing the interruption of an infrared light beam when flies are walking in horizontal tubes⁴¹. As shown in **Fig. 6f****,** *sky*^{*1*/*2*} mutants expressing Sky with positive pocket mutations appear restless and are much more active throughout the 24 h cycle compared to *sky*^{*1*/*2*} mutants that express wild type Sky. Taken together, the data indicate that positive pocket mutations in Sky cause hyper activity, increased temperature sensitivity and seizures in fruit flies.

### 8. Epilepsy-like defects caused by Sky cationic pocket mutations are rescued by partial loss of synaptojanin

Our findings underpin the importance of the cationic phosphoinositide-binding pocket for Sky function in the nervous system. We therefore tested if genetically increasing synaptic PI(4,5)P₂ levels *in vivo* can rescue the defects associated with the Skywalker cationic pocket mutants. For this purpose we used mutations in *synaptojanin* that encodes a synapse-specific phosphoinositide phosphatase^{25,26,42}. Compared to controls *synaptojanin* mutants harbor increased PI(4,5)P₂ levels at synapses⁴³. We find that heterozygous *synaptojanin* mutants very significantly rescue the cellular and behavioral defects associated with Sky positive pocket mutants (**Fig. 7**). *Sky*^{*1*/*2*} mutants expressing Sky^{R79C} and heterozygous for *synaptojanin* (*synj*^{+/-}) show normal GFP Sky^{R79C} mobility in our FRAP assay, indicating that increasing the PI(4,5)P₂ levels restricts the diffusion of the GFP-Sky^{R79C} mutant at synapses (**Fig. 7a****, b**). Furthermore, while expression of the Sky^{R79C} mutant in *sky*^{*1*/*2*} flies could only partially reduce the number of FM 1-43 accumulations, removing one copy of *synj* rescues the FM 1-43 accumulation defect, and no significant difference from controls (*yw; FRT40A*) or *sky*^{*1*/*2*} mutants expressing wild type Sky^{WT} is observed (**Fig. 7c****, d**). Finally, *sky*^{*1*/*2*} mutants expressing Sky^{R79C} in heterozygous *synaptojanin* mutants do not display a significant sensitivity to temperature (**Fig. 7e**) and show significantly fewer seizures (**Fig. 7f**).

Hence, partial decrease in Synaptojanin activity is sufficient to rescue the defects associated with disruption of the cationic pocket in Skywalker.

### 9. Epilepsy-like defects caused by Sky cationic pocket mutations are rescued by treatment with a PI(4,5)P₂ stimulating compound, the alkyl phospholipid Miltefosine, in fruit flies

To assure that stimulation of PI(4,5)P₂ levels *in vivo* can rescue the defects associated with the Skywalker cationic pocket mutants (Sky^{R79C}), a commercially available alkyl phospholipid compound, Miltefosine, with known impact on PI(4,5)P₂ levels was tested for treatment of gender selected adult 4-7 day old *sky*^{*1*/*2*} flies with cationic pocket mutations. For drug feeding, 10-20 flies per treatment vial were maintained in a 12 h light and dark cycle to maintain circadian rhythm, while the analysis was performed on 5 flies per vial for flight, after acclimatization to the test chamber, and 10 flies per vial for stimulation or behavioural challenge by vortexing. In total n= 20 flies were analyzed per treatment as well as for the control flies reared on normal food (mock-treated). The effect of Miltefosine (Sigma, M5571) daily administered in dyed food at 50 µg/mL or at 100 µg/mL was tested over a feeding period of 2 and 8 days of drug exposure, after which animal behaviour was analyzed via flight and seizure assays.

Compared to mock-treated controls, we found a significant rescue of the cellular and behavioural defects associated with Sky positive pocket mutants for Miltefosine treatment at 50 µg/mL, but not at 100 µg/mL, probably due to toxicity at the highest concentration (Figure 8). The rescue of seizures was highly significant (***, p<0.001) after 2 days of treatment at 50 µg/mL and lasted at least up to 8 days after treatment (*, p<0.1) (Figure 8). Finally, the compound treatments of *sky*^{*1*/*2*} mutants expressing Sky^{R79C} resulted in significantly fewer seizure-like episodes following arousal stimulation. Hence, increasing the PI(4,5)P₂ level is sufficient to rescue the defects associated with disruption of the cationic pocket in Skywalker.

### 10. Rescue treatment in fly using alkylphospholipid compounds.

Perifosine (Sigma Aldrich, SML0612), Edelfosine (Sigma Aldrich, SML0332) and Erufosine (6d cpd, Alam et al., 2012. European J. Med. Chem. 47: 485-492.) also belong to the same class of PI(4,5)P₂ stimulating compounds annotated as alkyl phospholipids, as Miltefosine is. Erufosine has furthermore been described as having the potential to cross the blood-brain-barrier (Van Blitterswijk and Verheij, 2013. Biochim. & Biophys. Acta, 1831:663-674). To confirm that alkyl phospholipids can rescue the defects associated with the Skywalker cationic pocket mutants (Sky^{R79C}), those compounds have been incorporated in fly testing, by quantifying their seizure and flight activity.

Each data point represents averages of 10 animals tested over 8 days and analyzed per drug treatment as well as the control flies reared on normal food (mock-treated).

For drug treatment, a concentration range between 100 and 1000 µg/mL was tested for daily administration. A positive effect was observed for Perifosine daily administered in dyed food at a concentration of 1000 µg/mL, and for Erufosine daily administered in dyed food at a concentration between 400-1000 µg/mL. For Edelfosine, the treatment at a concentration of 500 µg/mL is shown in Figure 9B and oral uptake of the drug results in a decrease of the number of seizures, albeit not at significantly different levels as compared to the control. Treatments were performed and were tested over a feeding period of 2 and 8 days of drug exposure, after which animal behaviour was tested in flight and seizure assays.

Compared to mock-treated controls, we found a significant rescue of seizures, which are associated with Sky positive pocket mutants, for the alkyl phospholipid compounds after 8 days of treatment, although at different concentrations of administration (Figure 9). Hence, increasing the PI(4,5)P₂ level via treatment with alkyl phospholipid treatment is sufficient to rescue the defects associated with disruption of the cationic pocket in Skywalker.

### 11. In vitro screening assay for inhibition of the 5'-phosphatase domain of human Syni1

With the aim to *in vitro* screen compound libraries for potent Synj1 inhibitors specifically affecting the 5'-phosphatase domain activity, a robust enzymatic assay was used. First, an expression plasmid for the recombinant expression of the 5'-phosphatase domain of Synj1 (SEQ ID NO:1) was constructed. This protein was subsequently produced in high amounts and purified to homogeneity. A commercially available assay in a 96-well plate format is the Malachite Green Phosphate Assay Kit (BioAssay Systems).

Initial experiments using the 5'-phosphatase domain of Synj1 and inositol-3-phosphate (IPs) (Merck Millipore) as substrate showed clear 5'-phosphatase activity using the Malachite Green Phosphate assay according to the manufacturer's protocol. Moreover, addition of the inhibitor YU144118⁶⁵ showed inhibition of the activity in a dose dependent way. After assay optimization, the assay is used to measure inhibitory activity of known compounds as well as to further allow a high-throughput setup for compound screening.

A number of compounds were analyzed to test their effect on the phosphatase activity of the 5'-phosphatase domain specifically. YU144118, as well as several alkyl phospholipids (Miltefosine, Perifosine and Edelfosine) were analyzed for their inhibitory activity in this assay. As shown in Figure 10, after background correction, those compounds can be considered as effective inhibitors of phosphatase activity in the Malachite Green assay.

### Discussion

Our biochemical and structural data show that Sky preferentially binds phosphoinositides that are phosphorylated in the 4- and 5-position, including PI(4,5)P₂ and PI(3,4,5)P₃. Similar to other phosphoinositide binding domains, the phosphoinositide-binding pocket of Sky is constituted primarily of arginine and lysine residues that interact via salt bridges with the phosphate groups and form a relatively shallow binding pocket. However, in contrast to the majority of previously characterized phosphoinositide-binding domains, the positively charged residues in the Sky TBC domain are provided by secondary structure elements from different regions in the amino acid sequence, such that a phosphoinositide-binding motif cannot be easily discerned from the primary sequence alone⁴⁸. The binding specificity of Sky for 4- and 5-phosphorylated phosphoinositides, including PI(4,5)P₂ and PI(3,4,5)P₃, agrees with the physiological role of Sky in vesicular trafficking. Sky has been proposed to orchestrate the trafficking of synaptic vesicles at nerve terminals^{20,24,30}, a process that requires the correct targeting of the protein to endocytic membranes thereby enabling local interactions to guide vesicle trafficking. Phosphoinositides phosphorylated at specific locations on the inositol ring serve as identity tags for specific membrane compartments, recruiting specific phosphoinositide-binding proteins^{31,50}. PI(4,5)P2 and PI(3,4,5)P₃ account for about 1% of the phospholipids in the inner leaflet of the plasma membrane and concentrate in endocytic and exocytic zones during synaptic vesicle cycling, allowing synaptic factors to bind and orchestrate vesicle formation and fusion^{37,51}. Sky localizes to synapses²⁰ and we show that mutations in the phosphoinositide-binding pocket significantly increase the mobility of Sky. These results, together with our observations that Sky binds lipid-membranes, suggest that the wild type protein associates with membranes *in vivo.* We propose that Sky is recruited to the synaptic membrane zones that are rich in PI(4,5)P₂ and PI(3,4,5)P₃. Binding at these membrane compartments can potentially be stabilized further by interactions with other membrane-bound partner proteins. Conversely, binding of Sky to the membrane could also recruit and regulate target GTPases or other regulators. These (transient) interactions can then potentially participate in regulating Skywalker-dependent synaptic vesicle trafficking. Given that PI(4,5)P₂ and PI(3,4,5)P₃ are thought to be acutely produced during endo- and exocytic cycles, this model also implies that Sky can be dynamically engaged during specific vesicle trafficking steps.

The behavioural deficits seen upon expression of the clinical mutants correlate with synaptic vesicle trafficking defects that we also observed in loss-of-function sky mutants²⁰, indicating the clinical mutants also cause loss of a sky/TBC1D24 function. The defects in the pathogenic mutants are less severe than those seen in *sky*^{*1*/*2*} mutants, indicating they are partial loss of function alleles. This correlates with the observation that the pathogenic mutations in the phosphoinositide binding pocket significantly reduce the affinity for IP3 and PI(4,5)P₂, while they do not completely abolish binding. Moreover, the *in vivo* phenotypes might also be alleviated to a certain extent because the mutants are over expressed, driving the mutant protein to bind membranes. It is conceivable that these defects in membrane targeting of the clinical *Sky* mutants will in turn affect the interactions with partner proteins at the correct membrane compartments, eventually leading to trafficking defects.

### Materials and Methods

### 1. Cloning, Protein Expression and Protein Purification

For the structural and biochemical analyses the TBC domain of Sky (Sky1-353) was expressed from the pET28a vector (Novagen) as an N-terminal His6-tag fusion. All point mutations were created using QuickChange mutagenesis with *Pfu* high-fidelity DNA polymerase (Thermo Fisher Scientific). Wild-type and mutant proteins were expressed in E. *coli* C41 (DE3) cells and purified by immobilized metal ion affinity chromatography and size exclusion chromatography.

### 2. Structure determination

Crystals of Sky1-353 were obtained by the sitting drop vapor diffusion method at 277 K. Crystals of native and SeMet-labeled proteins were obtained in the presence of 20% PEG 3350 and 0.2 M ammonium citrate tribasic pH 7.0. The complex of Sky1-353 with IP3 was obtained by soaking crystals grown in 25% PEG 1500 and 0.1 M succinate/phosphate/glycine pH 7.0 with 5 mM IP3 (Merck Millipore) for 1 h. Before flash freezing in liquid nitrogen all crystals were briefly soaked in mother liquor supplemented with 25% glycerol.

Data were collected at 100 K at the Proxima 2 beamline of the SOLEIL synchrotron for native and SeMet-labeled protein, and at the ID23-1 beamline of the ESRF synchrotron for Sky1-353 in complex with IP3. Diffraction data were integrated and scaled with the program XDS⁵². The SeMet-labelled Sky1-353 structure was solved using the single-wavelength anomalous diffraction (SAD) method at the SeMet peak wavelength using Autosol and Autobuild from the Phenix suite⁵³. This initial low resolution model was then used to solve the structure of the high resolution native Sky1-353 structure using molecular replacement with Phaser54. The latter structure was used to solve the Sky1-353-IP3 complex with molecular replacement. Models were then improved by iterative cycles of refinement with Phenix and manual building in Coot⁵⁵. MolProbity was used for structure validation⁵⁶. X-ray data collection and refinement statistics are listed in **Table 1.**

### 3. Lipid binding and thermal shift assays

Liposomes were prepared using either Folch brain total lipid extract or using a mix of phosphatidylcholine and phosphatidylserine (80:20 ratio) supplemented with 0.5%, 2% or 5% of a specific phosphoinositide as indicated. The liposomes were subsequently incubated with 6 µg of wild-type or mutant Sky1-353. PI(4,5)P₂-Grip (Echelon Biosciences, Salt Lake City, USA) was used as a positive control. Subsequently, this mix was loaded on a sucrose cushion (30 % sucrose layer, 25% sucrose layer and HP30 buffer layer) and centrifuged. Individual fractions from the top to the bottom were collected and assayed using Western blotting against His6 (Sky) or GST (PI(4,5)P₂-Grip).

Titration curves for binding of IP3 to Sky1-353 were obtained via thermal shift assays at different concentrations of IP3. Thermal unfolding was detected by following SYPRO orange fluorescence using a CFX connect Real-Time PCR System (BioRad). 0.2 mg/ml (4.7 µM) protein was combined with 3X SYPRO Orange Protein Gel Stain (Thermo Fisher Scientific) at different concentrations of MgSO4 and IP3. The temperature was increased from 20°C to 80°C at 0.5°C/30s steps. All measurements were done in triplicate. Plots of Tm versus IP3 or MgSO₄ concentration were fitted on a quadratic equation.

### 4. Genetics

Fly stocks were reared on standard corn meal and molasses medium at 21 °C. For experiments, mutants and controls were grown in optimal conditions on grape juice plates with fresh yeast paste. *sky* mutants are *yw eyFLP; FRT40A sky1* / *FRT40A sky2* and wild type controls are *yw eyFLP; FRT40A* as previously described20. *Synaptojanin* mutants are *eyFLP; FRT42D synj1* as previously described²⁶ and the *sky1* allele (on 2L) was recombined with the *synj1* allele (on 2R). The presence of *FRT40A* or *FRT42D* was not verified. nSybGal4 is from the Bloomington *Drosophila* stock center. Wild type *sky* cDNA, patient mutation alleles and triple mutations in the positive pocket were based on BDGP cDNA clone LD10117 and were synthesized from three overlapping DNA blocks (Integrated DNA Technologies) with overlapping ends and a pUAST-attB vector backbone⁵⁷. The second DNA block was adapted to include either a patient single mutation or triple point mutations. DNA blocks and the pUAST-attB vector were digested with Notl and Xhol restriction enzymes and ligated via Gibson Assembly⁵⁸ (New England Biolabs, Ipswich, USA), and correct synthesis and ligation confirmed by sequencing. UAS-Sky and all Sky mutants were inserted into the genome using ϕC31-mediated integration at the VK33 (cytological location 65B2, PBac{yellow+-attP-3B}VK00033) docking site on the third chromosome. Primers for UAS-Sky cDNA amplification are: for: 5'agaattcATGCCATACCATCGAGGCGG3'; rev: 5'aagcggccgcTTAGATGCCCACGAATCCGTAG3' (EcoRI and Notl restriction sites in lower case).

### 5. FRAP Assay

*Sky*^{1/2}larvae overexpressing the Sky cDNAs GFP-SkyWT, GFP-SkyR79C, GFP-SkyR281C, GFPSky3Glu driven by nSybGal4 were dissected in HL-3 (in mM: NaCl (110), KCI (5), NaHCOs (10), HEPES (5), sucrose (30), trehalose (5), MgCl₂ (10) pH 7.2), segmental nerves were cut and NMJ boutons of muscles 6/7 were imaged on a Nikon A1R confocal microscope and 60x 1.0 NA water immersion lens at 1 fps. For photobleaching a region of 20% of the bouton surface was selected and stimulated in a single scan step at λ= 405nm (90.1%) and 488 nm (99.8%) for < 1 s. The recovery of fluorescence in the bleached region was measured over the course of 1 min at room temperature using standard GFP optics. The time constant τ of the recovery curve was determined for each genotype. We also assessed the photo-recovery of Synaptotagmin-GFP (Syt), a protein that is always associated with vesicle membranes (Syt is a transmembrane protein).

### 6. FM 1-43 dye uptake

Third instar larvae were dissected in HL-3 and motor neurons were cut. Stimulation in the presence of FM 1-43 (4 µM) (Invitrogen), for 1 min was performed using HL-3 with 90 mM KCI: (mM) NaCl (25), KCI (90), NaHCOs (10), HEPES (5), sucrose (30), trehalose (5), MgCl₂ (10), CaCl₂ (1.5), pH 7.2. Non-internalized dye was removed by washing with HL-3. FM 1-43 at two muscle 6/7 NMJ was recorded with a Nikon A1R confocal microscope and 60x 1.0 NA water immersion lens at room temperature utilizing λ= 488 nm excitation and a 510/20 nm band pass emission filter.

### 7. Transmission Electron Microscopy

Third instar larvae were dissected in HL-3 and motor neurons cut. Preparations were stimulated for 1 min in HL-3 with KCI (60 mM KCI and 1.5 mM CaCl₂), followed by fixation (1 % glutaraldehyde, 4 % paraformaldehyde, 1 mM MgCl₂ in 0.1 M Na-cacodylate buffer, pH 7.4). Subsequently, larvae were osmicated in OsO₄/Na-cacodylate for 2 h and stained in 2 % aqueous uranyl acetate for 1.5 h. Specimens were dehydrated in a graded ethanol series and embedded in Agar 100 (Laborimpex, Agar Scientific). Ultrathin sections (60-70 nm) were collected on butvar coated grids (Laborimpex, Agar Scientific). Grids were recorded with a JEM1400 (JEOL) transmission electron microscope at 80 kV. To analyze synaptic boutons, ultrastructural profile areas and synaptic vesicle content were quantified in imaged (NIH).

### 8. Animal Behaviour

Adult flies of the genotype *sky¹* / *sky² (sky*^{*1*/}*²);* nsyb-Gal4 / UAS-Sky^{R79C} or genotypes as indicated, were raised at 21°C on standard fly food, containing the indicated drugs (Miltefosine, Perifosine, Edelfosine, Erufosine) or on control food prepared without drugs. Animals were collected and gender selected 2 to 5 days after enclosure. On days 2 and 8 of incubation on drugs, flight assays were performed with groups of 5 gender-selected animals in each transparent vial. Following a tap to collect the animals at the bottom of the vial, behaviour was distinguished between walking, jumping and flight. Seizure assays were performed with groups of 10 gender-selected animals that were transferred into transparent vials and excited by vortexing once for 10 seconds in behaviour studies, or in a sequence (1 minute vortexing, 1 minute rest, 1 minute vortexing) for drug treatment studies. This test sequence was repeated following 5 minutes of rest. Active and fallen flies were quantified over the course of the following minute. To ensure natural behaviour animals were not anesthetized on the day of experiments or over the course of drug application.

### 9. Biochemical screening assay for Synj1 5'-phosphatase domain-specific inhibitors.

The Malachite Green Phosphate Assay Kit (BioAssay Systems) is a discontinuous assay that measures the concentrations of enzymatically released phosphate after formation of a complex with malachite green and molybdate (absorbance is measured at 620 nm). The assay optimized for screening of inhibitory compounds was used according to manufacturers protocol, with the 5'-phosphatase domain of Synj1 (200 nM HIS-tagged human SYNJ1 amino acid 528-873 (hSYNJ1 corresponds to SEQ ID NO:1)) as enzyme, and inositol-3-phosphate (40 µM IPs) (Merck Millipore) as substrate, in buffer 25 mM HEPES pH 7.5, 150 mM NaCl, 5 % glycerol, and 1mM DTT. As a read-out the Absorbance at 620 nm after 60 min incubation is measured and the background starting signal is subtracted. For inhibitory compound screening, 1mM of the test compound dissolved in 1% DMSO was used.

### REFERENCES

1. Corbett, M. et al. A focal epilepsy and intellectual disability syndrome is due to a mutation in TBC1D24. Am. J. Hum. Genet. 87, 371-5 (2010).
2. Falace, A. et al. TBC1D24, an ARF6-interacting protein, is mutated in familial infantile myoclonic epilepsy. Am. J. Hum. Genet. 87, 365-70 (2010).
3. Guven, A. & Tolun, A. TBC1D24 truncating mutation resulting in severe neurodegeneration. J. Med. Genet. 50, 199-202 (2013).
4. Milh, M. et al. Novel compound heterozygous mutations in TBC1D24 cause familial malignant migrating partial seizures of infancy. Hum. Mutat. 34, 869-72 (2013).
5. Muona, M. et al. A recurrent de novo mutation in KCNC1 causes progressive myoclonus epilepsy. Nat. Genet. 47, (2014).
6. Poulat, A.-L. et al. Homozygous TBC1D24 mutation in two siblings with familialinfantile myoclonic epilepsy (FIME) and moderate intellectual disability. Epilepsy Res.111, 72-77 (2015).
7. Azaiez, H. et al. TBC1D24 mutation causes autosomal-dominant nonsyndromic hearing loss. Hum. Mutat. 35, 819-823 (2014).
8. Bakhchane, A. et al. Recessive TBC1 D24 Mutations Are Frequent in Moroccan Non-Syndromic Hearing Loss Pedigrees. PLoS One 10, e0138072 (2015).
9. Stražišar, B. G., Neubauer, D., Paro Panjan, D. & Writzl, K. Early-onset epileptic encephalopathy with hearing loss in two siblings with TBC1D24 recessive mutations. Eur. J. Paediatr. Neurol. 19, 251-256 (2015).
10. Zhang, L., Hu, L., Chai, Y. & Pang, X. A Dominant Mutation in the Stereocilia expressing Gene TBC1D24 is a Probable Cause for Non-syndromic Hearing Impairment. Hum. Mutat. 1-14 (2014). doi:10.1002/humu.22558.
11. Doummar, D. et al. A Novel Homozygous TBC 1 D 24 Mutation Causing Multifocal Myoclonus With Cerebellar Involvement. Mov. Disord. 30, 1431-1432 (2015).
12. Campeau, P. M. & Hennekam, R. C. DOORS syndrome: Phenotype, genotype and comparison with Coffin-Siris syndrome. Am. J. Med. Genet. C. Semin. Med. Genet. 166, 327-32 (2014).
13. Campeau, P. M. et al. The genetic basis of DOORS syndrome: an exome-sequencing study. Lancet Neurol. 13, 44-58 (2014).
14. Mucha, B. E., Hennekam, R. C., Sisodiya, S. & Campeau, P. M. TBC1D24-Related Disorders. (2015). at <http://www.ncbi.nlm.nih.gov/books/NBK274566/>
15. Frasa, M. a M., Koessmeier, K. T., Ahmadian, M. R. & Braga, V. M. M. Illuminating the functional and structural repertoire of human TBC/RABGAPs. Nat. Rev. Mol. Cell Biol. 13, 67-73 (2012).
16. Stenmark, H. Rab GTPases as coordinators of vesicle traffic. Nat. Rev. Mol. Cell Biol. 10, 513-25 (2009).
17. Fukuda, M. TBC proteins: GAPs for mammalian small GTPase Rab? Biosci. Rep. 31, 159-168 (2011).
18. Gavriljuk, K. et al. Catalytic mechanism of a mammalian Rab·RabGAP complex in atomic detail. Proc. Natl. Acad. Sci. U. S. A. 109, 21348-53 (2012).
19. Pan, X., Eathiraj, S., Munson, M. & Lambright, D. G. TBC-domain GAPs for Rab GTPases accelerate GTP hydrolysis by a dual-finger mechanism. Nature 442, 303-6 (2006).
20. Uytterhoeven, V., Kuenen, S., Kasprowicz, J., Miskiewicz, K. & Verstreken, P. Loss ofskywalker reveals synaptic endosomes as sorting stations for synaptic vesicle proteins. Cell 145, 117-32 (2011).
21. Chaineau, M., loannou, M. S. & McPherson, P. S. Rab35: GEFs, GAPs and effectors. Traffic 14, 1109-17 (2013).
22. Cherfils, J. Arf GTPases and their effectors: assembling multivalent membrane-binding platforms. Curr. Opin. Struct. Biol. 29, 67-76 (2014).
23. Chesneau, L. et al. An ARF6/Rab35 GTPase cascade for endocytic recycling and successful cytokinesis. Curr. Biol. 22, 147-153 (2012).
24. Fernandes, A. C. et al. Reduced synaptic vesicle protein degradation at lysosomes curbs.J. Cell Biol. 207, 453-462 (2014).
25. Cremona, O. et al. Essential role of phosphoinositide metabolism in synaptic vesiclerecycling. Cell 99, 179-88 (1999).
26. Verstreken, P. et al. Synaptojanin is recruited by endophilin to promote synaptic vesicle uncoating. Neuron 40, 733-48 (2003).
27. Park, S.-Y., Jin, W., Woo, J. R. & Shoelson, S. E. Crystal structures of human TBC1D1 and TBC1D4 (AS160) RabGTPase-activating protein (RabGAP) domains reveal critical elements for GLUT4 translocation. J. Biol. Chem. 286, 18130-8 (2011).
28. Ashkenazy, H., Erez, E., Martz, E., Pupko, T. & Ben-Tal, N. ConSurf 2010: calculating evolutionary conservation in sequence and structure of proteins and nucleic acids. Nucleic Acids Res. 38, W529-W533 (2010).
29. Rehman, A. U. et al. Mutations in TBC1D24, a gene associated with epilepsy, also cause nonsyndromic deafness DFNB86. Am. J. Hum. Genet. 94, 144-152 (2014).
30. Falace, A. et al. TBC1D24 regulates neuronal migration and maturation through modulation of the ARF6-dependent pathway. Proc. Natl. Acad. Sci. U. S. A. 111, 2337-42 (2014).
31. Di Paolo, G. & De Camilli, P. Phosphoinositides in cell regulation and membrane dynamics. Nature 443, 651-7 (2006).
32. Czech, M. P. PIP2 and PIP3: complex roles at the cell surface. Cell 100, 603-6 (2000).
33. Khuong, T. M. et al. Synaptic PI(3,4,5)P3 is required for Syntaxin1A clustering and neurotransmitter release. Neuron 77, 1097-108 (2013).
34. Niesen, F. H., Berglund, H. & Vedadi, M. The use of differential scanning fluorimetry to detect ligand interactions that promote protein stability. Nat. Protoc. 2, 2212-2221 (2007).
35. Bottomley, M. J., Salim, K. & Panayotou, G. Phospholipid-binding protein domains. Biochim. Biophys. Acta - Mol. Cell Biol. Lipids 1436, 165-183 (1998).
36. Bravo, J. et al. The crystal structure of the PX domain from p40(phox) bound to phosphatidylinositol 3-phosphate. Mol. Cell 8, 829-39 (2001).
37. Saheki, Y. & De Camilli, P. Synaptic Vesicle Endocytosis. Cold Spring Harb. Perspect. Biol. 4, a005645-a005645 (2012).
38. Salkoff, L. & Kelly, L. Temperature-induced seizure and frequency-dependent neuromuscular block in a ts mutant of Drosophila. Nature 273, 156-8 (1978).
39. Titus, S. A., Warmke, J. W. & Ganetzky, B. The Drosophila erg K+ channel polypeptide is encoded by the seizure locus. J. Neurosci. 17, 875-81 (1997).
40. Krans, J. L., Rivlin, P. K. & Hoy, R. R. Demonstrating the temperature sensitivity of synaptic transmission in a Drosophila mutant. J. Undergrad. Neurosci. Educ. 4, A27-33 (2005).
41. Rosato, E. & Kyriacou, C. P. Analysis of locomotor activity rhythms in Drosophila. Nat. Protoc. 1, 559-68 (2006).
42. Harris, T. W., Hartwieg, E., Horvitz, H. R. & Jorgensen, E. M. Mutations in synaptojanin disrupt synaptic vesicle recycling. J. Cell Biol. 150, 589-600 (2000).
43. Verstreken, P. et al. Tweek, an evolutionarily conserved protein, is required for synaptic vesicle recycling. Neuron 63, 203-15 (2009).
44. D'Souza-Schorey, C. & Chavrier, P. ARF proteins: roles in membrane traffic and beyond. Nat. Rev. Mol. Cell Biol. 7, 347-58 (2006).
45. Chavrier, P. & Goud, B. The role of ARF and Rab GTPases in membrane transport. Curr. Opin. Cell Biol. 11, 466-75 (1999).
46. Wittinghofer, A. & Vetter, I. R. Structure-function relationships of the G domain, a canonical switch motif. Annu. Rev. Biochem. 80, 943-71 (2011).
47. Sklan, E. H. et al. TBC1D20 is a Rab1 GTPase-activating protein that mediates hepatitis C virus replication. J. Biol. Chem. 282, 36354-61 (2007).
48. Moravcevic, K., Oxley, C. L. & Lemmon, M. A. Conditional Peripheral Membrane Proteins: Facing up to Limited Specificity. Structure 20, 15-27 (2012).
49. Kutateladze, T. G. Translation of the phosphoinositide code by PI effectors. Nat. Chem. Biol. 6, 507-513 (2010).
50. Lemmon, M. a. Membrane recognition by phospholipid-binding domains. Nat. Rev. Mol. Cell Biol. 9, 99-111 (2008).
51. Slabbaert, J. R., Khuong, T. M. & Verstreken, P. Phosphoinositides at the neuromuscular junction of Drosophila melanogaster: a genetic approach. Methods Cell Biol. 108, 227-47 (2012).
52. Kabsch, W. Xds. Acta Crystallogr. D. Biol. Crystallogr. 66, 125-32 (2010).
53. Adams, P. D. et al. PHENIX: A comprehensive Python-based system for macromolecular structure solution. Acta Crystallogr. Sect. D Biol. Crystallogr. 66, 213-221 (2010).
54. McCoy, A. J. et al. Phaser crystallographic software. J. Appl. Crystallogr. 40, 658-674 (2007).
55. Emsley, P. & Cowtan, K. Coot: model-building tools for molecular graphics. Acta Crystallogr. D. Biol. Crystallogr. 60, 2126-32 (2004).
56. Chen, V. B. et al. MolProbity: all-atom structure validation for macromolecular crystallography. Acta Crystallogr. Sect. D Biol. Crystallogr. 66, 12-21 (2010).
57. Bischof, J., Maeda, R. K., Hediger, M., Karch, F. & Basler, K. An optimized transgenesis system for Drosophila using germ-line-specific phiC31 integrases. Proc. Natl. Acad. Sci. U. S. A. 104, 3312-7 (2007).
58. Gibson, D. G. et al. Enzymatic assembly of DNA molecules up to several hundred kilobases. Nat. Methods 6, 343-5 (2009).
59. Roy, A., Skibo, J., Kalume, F., Ni, J., Rankin, S., Lu, Y., Dobyns, W.B., Mills, G.B., Zhao, J.J., Baker, S.J., and Mille, K.J. Mouse models of human PIK3CA-related brain overgrowth have acutely treatable epilepsy. eLife 2015;4:e12703. DOI: 10.7554/eLife.12703
60. Hardies, K., Cai, Y., Jardel, C., Jansen, A.C., Cao, M., May, P., Djémié, T., Hachon Le Camus, C., Keymolen, K., Deconinck, T., Bhambhani, V., Long, C., Sajan, S.A., Helbig, K.L., Suls, A., Balling, R., Helbig, I., De Jonghe, P., Depienne, C., De Camilli, P., and Weckhuysen S. Loss of SYNJ1 dual phosphatase activity leads to early onset refractory seizures and progressive neurological decline. Brain, July 2016. DOI: http://dx.doi.org/10.1093/brain/aww180
61. McIntire, L.B.J, Lee, K.I., Chang-Ileto, B., Di Paolo, G., and Kim, T-W. 2014. Screening assay for small molecule inhibitors of synaptojanin 1, a synaptic phosphoinositide phosphatase. J Biomol Screen. 19(4): 585-594.
62. Muscolini, M., Camperio, C., Capuano, C., Caristi, S., Piccolella, E., Galandrini, R., and Tuosto, L. 2013. Phosphatidylinositol 4-Phosphate 5-Kinase a Activation Critically Contributes to CD28-Dependent Signaling Responses.J. Immunol. 190:5279-5286.
63. Ding, Y., Li, J., Enterina, J.R., Shen, Y., Zhang, I., Tewson, P.H., Mo, GCH., Zhang, J., Quinn, A-M., Hughes, T.E., Maysinger, D., Alford, S.C., Zhang, Y., & Campbell, R.E. 2015. Ratiometric biosensors based on dimerization-dependent fluorescent protein exchange. Nature methods. 12(3): 195-201.
64. Lee, S.Y., Wenk, M.R., Kim, Y., Nairn, A.C., and De Camilli P. 2004. Regulation of Synaptojanin 1 by cyclin-dependent kinase 5 at synapses. Proc. Natl. Acad. Sci. U. S. A 101(2): 546-551.
65. Pirruccello, M., Nandez, R., Idevall-Hagren, O., Alcazar-Roman, A., Abriola, L., Berwick, S.A., Lucast, L., Morel, D. and De Camilli, P. 2014. Identification of Inhibitors of Inositol 5-Phosphatases through Multiple Screening Strategies. ACS Chem. Biol., 9, 1359-1368.
66. Varnai, P., and Balla, T. 2009. Live cell imaging of phosphoinositide dynamics with fluorescent protein domains. Bioch. Biophys. Acta 1761: 957-967.
67. Nelson, C.P., Nahorski, S.R., and Challiss, R.A.J. 2008. Temporal profiling of changes in phosphatidylinositol 4,5-bisphosphate, inositol 1,4,5-trisphosphate and diacylglycerol allows comprehensive analysis of phospholipase C-initiated signalling in single neurons. J. Neurochem. 107: 602-615.
68. Sharma, V.P., Desmarais, V., Sumners, C., Shaw, G., and Narang, A. 2008. Immunostaining evidence for PI(4,5)P2 localization at the leading edge of chemoattractant-stimulated HL-60 cells. J. Leuk. Biol. 84: 440-447.
69. Dillon, C., and Goda, Y. (2005). The Actin Cytoskeleton: Integrating Form and Function at the Synapse. Annu. Rev. Neurosci. 28: 25-55.

## Claims

1. A phosphoinositide phosphatase inhibitor, which increases PI(4,5)P₂ levels in a cell, for use in treatment of TBC1 D24-associated disorders.

2. The phosphoinositide phosphatase inhibitor according to claim 1, for use in treatment of TBC1 D24-associated disorders, wherein said TBC1 D24-associated disorders comprise severe forms of epilepsy (early onset epilepsy, familial infantile myoclonic epilepsy (FIME), focal epilepsy, dysarthria, myoclonic epilepsy with dystonia, familial malignant migrating partial seizures of infancy, *De Flippo* malignant migrating partial seizures of infancy), variable degrees of intellectual disability, hearing loss (nonsyndromic deafness, autosomal-dominant nonsyndromic hearing loss, and dominant nonsyndromic hearing impairment), cortical myoclonus and cerebellar ataxia, and DOORS syndrome (deafness, onychodystrophy, osteodystrophy, mental retardation and seizures).

3. The phosphoinositide phosphatase inhibitor according to claims 1 or 2, for use in treatment of TBC1D24-associated disorders, wherein said phosphoinositide phosphatase is Synaptojanin-1.

4. The phosphoinositide phosphatase inhibitor according to claim 3, for use in treatment of TBC1D24-associated disorders, wherein said phosphoinositide phosphatase is the Synaptojanin-1 5' phosphatase.

5. The phosphoinositide phosphatase inhibitor according to any one of claims 1 to 4, for use in treatment of TBC1D24-associated disorders, wherein said PI(4,5)P₂ levels are increased in neuronal cells.

6. The phosphoinositide phosphatase inhibitor according to any one of claims 1 to 5, for use in treatment of TBC1D24-associated disorders, selected from the list consisting of bpV(pic), perifosine, edelfosine, miltefosine, erufosine, or YU144118, or a salt or hydrate derivative form thereof.

7. The phosphoinositide phosphatase inhibitor according to any one of claims 1 to 6, for use in treatment of TBC1D24-associated disorders, wherein said inhibitor is an alkyl phospholipid.

8. The phosphoinositide phosphatase inhibitor according to claim 7, for use in treatment of TBC1D24-associated disorders, wherein said inhibitor is miltefosine, or a salt or hydrate derivative form thereof.

9. The phosphoinositide phosphatase inhibitor according to claim 7, for use in treatment of TBC1D24-associated disorders, wherein said inhibitor is perifosine, or a salt or hydrate derivative form thereof.

10. The phosphoinositide phosphatase inhibitor according to claim 7, for use in treatment of TBC1D24-associated disorders, wherein said inhibitor is edelfosine, or a salt or hydrate derivative form thereof.

11. The phosphoinositide phosphatase inhibitor according to claim 7, for use in treatment of TBC1D24-associated disorders, wherein said inhibitor is erufosine, or a salt or hydrate derivative form thereof.

12. The phosphoinositide phosphatase inhibitor according to claim 6, for use in treatment of TBC1D24-associated disorders, wherein said inhibitor is YU144118, or a salt or hydrate derivative form thereof.

## Patentansprüche

1. Phosphoinositidphosphatase-Hemmer, der PI(4,5)P₂-Spiegel in einer Zelle erhöht, zur Verwendung bei der Behandlung von TBC1D24-assoziierten Erkrankungen.

2. Phosphoinositidphosphatase-Hemmer nach Anspruch 1 zur Verwendung bei der Behandlung von TBC1D24-assoziierten Erkrankungen, wobei die TBC1D24-assoziierten Erkrankungen schwere Formen von Epilepsie (frühkindliche Epilepsie, familiäre juvenile myoklonische Epilepsie (FIME), fokale Epilepsie, Dysarthrie, myoklonische Epilepsie mit Dystonie, familiäre maligne migrierende partielle Anfälle im Kindesalter, maligne migrierende partielle Anfälle im Kindesalter nach *De Filippo*), variable Grade der geistigen Behinderung, Hörverlust (nicht-syndromale Gehörlosigkeit, autosomal-dominanter nicht-syndromaler Hörverlust und dominante nicht-syndromale Hörstörung), kortikaler Myoklonus und zerebelläre Ataxie und DOORS-Syndrom (Gehörlosigkeit, Onychodystrophie, Osteodystrophie, geistige Zurückgebliebenheit und Anfälle) umfassen.

3. Phosphoinositidphosphatase-Hemmer nach den Ansprüchen 1 oder 2 zur Verwendung bei der Behandlung von TBC1 D24-assoziierten Erkrankungen, wobei die Phosphoinositidphosphatase Synaptojanin-1 ist.

4. Phosphoinositidphosphatase-Hemmer nach Anspruch 3 zur Verwendung bei der Behandlung von TBC1D24-assoziierten Erkrankungen, wobei die Phosphoinositidphosphatase die Synaptojanin-1,5`-phosphatase ist.

5. Phosphoinositidphosphatase-Hemmer nach einem der Ansprüche 1 bis 4 zur Verwendung bei der Behandlung von TBC1D24-assoziierten Erkrankungen, wobei die PI(4,5)P₂-Spiegel in neuronalen Zellen erhöht sind.

6. Phosphoinositidphosphatase-Hemmer nach einem der Ansprüche 1 bis 5 zur Verwendung bei der Behandlung von TBC1D24-assoziierten Erkrankungen, der aus der Liste bestehend aus bpV(pic), Perifosin, Edelfosin, Miltefosin, Erufosin oder YU144118 oder einer Salz- oder Hydratderivatform davon ausgewählt ist.

7. Phosphoinositidphosphatase-Hemmer nach einem der Ansprüche 1 bis 6 zur Verwendung bei der Behandlung von TBC1D24-assoziierten Erkrankungen, wobei der Hemmer ein Alkylphospholipid ist.

8. Phosphoinositidphosphatase-Hemmer nach Anspruch 7 zur Verwendung bei der Behandlung von TBC1D24-assoziierten Erkrankungen, wobei der Hemmer Miltefosin oder eine Salz- oder Hydratderivatform davon ist.

9. Phosphoinositidphosphatase-Hemmer nach Anspruch 7 zur Verwendung bei der Behandlung von TBC1D24-assoziierten Erkrankungen, wobei der Hemmer Perifosin oder eine Salz- oder Hydratderivatform davon ist.

10. Phosphoinositidphosphatase-Hemmer nach Anspruch 7 zur Verwendung bei der Behandlung von TBC1D24-assoziierten Erkrankungen, wobei der Hemmer Edelfosin oder eine Salz- oder Hydratderivatform davon ist.

11. Phosphoinositidphosphatase-Hemmer nach Anspruch 7 zur Verwendung bei der Behandlung von TBC1D24-assoziierten Erkrankungen, wobei der Hemmer Erufosin oder eine Salz- oder Hydratderivatform davon ist.

12. Phosphoinositidphosphatase-Hemmer nach Anspruch 6 zur Verwendung bei der Behandlung von TBC1D24-assoziierten Erkrankungen, wobei der Hemmer YU144118 oder eine Salz- oder Hydratderivatform davon ist.

## Revendications

1. Inhibiteur de phosphoinositide phosphatase, qui augmente les niveaux de PI(4,5)P₂ dans une cellule, destiné à être utilisé dans le traitement de troubles associés à TBC1D24.

2. Inhibiteur de phosphoinositide phosphatase selon la revendication 1, destiné à être utilisé dans le traitement de troubles associés à TBC1D24, dans lequel lesdits troubles associés à TBC1D24 comprennent des formes graves d'épilepsie (épilepsie précoce, épilepsie myoclonique infantile familiale (FIME), épilepsie focale, dysarthrie, épilepsie myoclonique avec dystonie, crises partielles migratoires malignes familiales de la petite enfance, crises partielles migratoires malignes *De Flippo* de la petite enfance), des degrés variables de handicap intellectuel, la perte d'audition (surdité non syndromique, perte d'audition non syndromique autosomique dominante, et déficience auditive non syndromique dominante), la myoclonie corticale et l'ataxie cérébelleuse, et le syndrome DOORS (surdité, onychodystrophie, ostéodystrophie, retard mental et crises).

3. Inhibiteur de phosphoinositide phosphatase selon la revendication 1 ou 2, destiné à être utilisé dans le traitement de troubles associés à TBC1D24, dans lequel ladite phosphoinositide phosphatase est la Synaptojanine-1.

4. Inhibiteur de phosphoinositide phosphatase selon la revendication 3, destiné à être utilisé dans le traitement de troubles associés à TBC1D24, dans lequel ladite phosphoinositide phosphatase est la Synaptojanine-1 5' phosphatase.

5. Inhibiteur de phosphoinositide phosphatase selon l'une quelconque des revendications 1 à 4, destiné à être utilisé dans le traitement de troubles associés à TBC1D24, dans lequel lesdits niveaux de PI(4,5)P₂ sont augmentés dans des cellules neuronales.

6. Inhibiteur de phosphoinositide phosphatase selon l'une quelconque des revendications 1 à 5, destiné à être utilisé dans le traitement de troubles associés à TBC1D24, sélectionné parmi la liste constituée de bpV(pic), la périfosine, l'édelfosine, la miltéfosine, l'érufosine, ou YU144118, ou un sel ou une forme dérivée d'hydrate de celui-ci.

7. Inhibiteur de phosphoinositide phosphatase selon l'une quelconque des revendications 1 à 6, destiné à être utilisé dans le traitement de troubles associés à TBC1D24, dans lequel ledit inhibiteur est un phospholipide d'alkyle.

8. Inhibiteur de phosphoinositide phosphatase selon la revendication 7, destiné à être utilisé dans le traitement de troubles associés à TBC1D24, dans lequel ledit inhibiteur est la miltéfosine, ou un sel ou une forme dérivée d'hydrate de celle-ci.

9. Inhibiteur de phosphoinositide phosphatase selon la revendication 7, destiné à être utilisé dans le traitement de troubles associés à TBC1D24, dans lequel ledit inhibiteur est la périfosine, ou un sel ou une forme dérivée d'hydrate de celle-ci.

10. Inhibiteur de phosphoinositide phosphatase selon la revendication 7, destiné à être utilisé dans le traitement de troubles associés à TBC1D24, dans lequel ledit inhibiteur est l'édelfosine, ou un sel ou une forme dérivée d'hydrate de celle-ci.

11. Inhibiteur de phosphoinositide phosphatase selon la revendication 7, destiné à être utilisé dans le traitement de troubles associés à TBC1D24, dans lequel ledit inhibiteur est l'érufosine, ou un sel ou une forme dérivée d'hydrate de celle-ci.

12. Inhibiteur de phosphoinositide phosphatase selon la revendication 6, destiné à être utilisé dans le traitement de troubles associés à TBC1D24, dans lequel ledit inhibiteur est YU144118, ou un sel ou une forme dérivée d'hydrate de celui-ci.
